(19) Europäisches Patentamt
European Patent Office
Office européen des brevets

(11) **EP 4 786 464 A1**

(12) **EUROPEAN PATENT APPLICATION**
published in accordance with Art. 153(4) EPC

(43) Date of publication:
**05.08.2026 Bulletin 2026/32**

(21) Application number: **24870842.2**

(22) Date of filing: **26.09.2024**

(51) International Patent Classification (IPC):
***C07D 403/04*** *(2006.01)* ***C07D 205/04*** *(2006.01)*
***C07D 471/04*** *(2006.01)* ***C07D 487/00*** *(2006.01)*
***A61K 31/4184*** *(2006.01)* ***A61K 31/397*** *(2006.01)*
***A61P 25/28*** *(2006.01)*

(52) Cooperative Patent Classification (CPC):
**A61K 31/397; A61K 31/4184; A61P 25/28; C07D 205/04; C07D 403/04; C07D 471/04; C07D 487/00**

(86) International application number:
**PCT/CN2024/121304**

(87) International publication number:
**WO 2025/067298 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 CN 202311273882**
**15.12.2023 CN 202311736403**

(71) Applicant: **Simcere Pharmaceutical Co., Ltd.**
**Nanjing, Jiangsu 210032 (CN)**

(72) Inventors:
- **YANG, Shengwei**
  **Nanjing, Jiangsu 210042 (CN)**
- **TANG, Feng**
  **Shanghai 201318 (CN)**
- **LIU, Le**
  **Nanjing, Jiangsu 210042 (CN)**
- **ZHAO, Chen**
  **Nanjing, Jiangsu 210042 (CN)**
- **ZHOU, Minyun**
  **Nanjing, Jiangsu 210042 (CN)**

(74) Representative: **V.O.**
**P.O. Box 87930**
**2508 DH Den Haag (NL)**

# (54) COMPOUND AS GLUTAMINE CYCLASE INHIBITOR

(57) Disclosed are a compound represented by formula (A-I) or a stereoisomer thereof or a pharmaceutically acceptable salt thereof as a glutamine cyclase inhibitor, a preparation method therefor, a pharmaceutical composition comprising the compound represented by formula (A-I) or the stereoisomer thereof or the pharmaceutically acceptable salt thereof, and a use of the compound represented by formula (A-I) or the stereoisomer thereof or the pharmaceutically acceptable salt thereof in preventing or treating diseases or conditions mediated by QPCT and/or QPCTL, including neurodegenerative diseases.

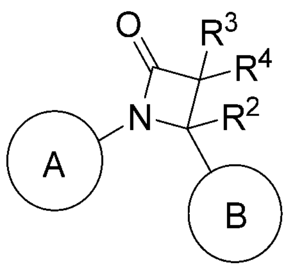

(A-I)

EP 4 786 464 A1

## Description

### CROSS-REFERENCE TO RELATED APPLICATIONS

The present application claims priority to and benefit of Chinese Patent Application No. 202311273882.4 filed with China National Intellectual Property Administration on September 27, 2023 and Chinese Patent Application No. 202311736403.8 filed with China National Intellectual Property Administration on December 15, 2023, the contents of which are incorporated herein by reference in their entirety.

### TECHNICAL FIELD

The present disclosure belongs to the technical field of pharmaceutics and particularly relates to a compound as a glutaminyl cyclase inhibitor, a preparation method therefor, a pharmaceutical composition comprising the compound, and use thereof in preventing or treating a disease or disorder mediated by QPCT and/or QPCTL.

### BACKGROUND

Alzheimer's disease (AD) is a neurodegenerative disease. It is characterized by irreversible functional impairment of the whole brain, which results in progressive decline in cognitive function, comprehension, and/or awareness.

The main histopathological markers of AD are Aβ plaques and neurofibrillary tangles caused by abnormal deposition of the β amyloid protein (Aβ) and Tau protein. According to a currently widely accepted theory, the initial pathological alterations in Alzheimer's disease are not caused by plaques, but rather by soluble Aβ aggregates, which are known as Aβ oligomers. Most Aβ peptides in the brains of AD patients are present in N-terminal cleaved and post-translationally modified forms. Glutaminyl cyclase (QC, also known as glutaminyl-peptide cyclotransferase QPCT) can catalyze post-translational chemical reactions of proteins or peptides, converting the N-terminal glutamine or glutamic acid residue into N-terminal pyroglutamic acid (pE) by releasing ammonia or water molecules, respectively. In neurodegenerative diseases, QPCT and its isozyme QPCTL (glutaminyl-peptide cyclotransferase-like) mediate the formation of pE-Aβ modifications in the brain. The hydrophobicity of Aβ is enhanced after the formation of pE at the N-terminus, and this form of Aβ exhibits higher neurotoxicity due to its stronger stability and hydrophobicity. In addition, the up-regulation of pro-inflammatory factors (cytokines and chemokines) is also a feature of the pathological process of AD. Among these pro-inflammatory factors, the chemokine CCL2 (also known as monocyte chemoattractant protein-1 (MCP-1)) plays an important role in the stimulation and over-activation of glial cells. Glutaminyl cyclase can also modify the N-terminal glutamine residue to form pE-CCL2. Such modification can render CCL2 resistant to degradation and mediate receptor activation. CCL2 and other chemokines can induce the expression of QC, thereby triggering a vicious cycle between pE-Aβ deposition and neuroinflammation. Therefore, inhibition of QC activity is a potential therapeutic strategy for treating pE-related diseases, including but not limited to Alzheimer's disease, cancer, and the like. In view of this, disclosed herein are a class of QPCT and/or QPCTL inhibitors with a novel structure, excellent efficacy, high bioavailability, and high potential to be manufactured as a drug.

### SUMMARY

In one aspect, the present disclosure provides a compound of formula (A-I) or a stereoisomer thereof or a pharmaceutically acceptable salt thereof,

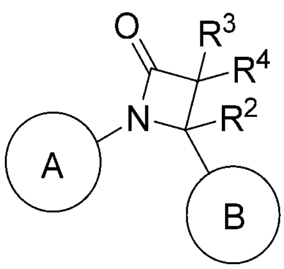

(A-I)

wherein

ring A is selected from

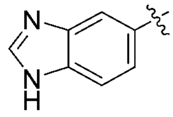 or 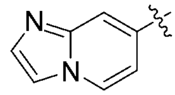 ,

wherein the

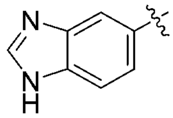

or

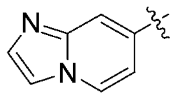

is optionally substituted with $R^5$;

$R^5$ is selected from D, halogen, CN, $NH_2$, OH, $C_1$-$C_6$ alkyl, $C_1$-$C_6$ alkoxy, $C_1$-$C_6$ alkylamino, $C_1$-$C_6$ haloalkyl, or $C_1$-$C_6$ deuterated alkyl;

ring B is selected from

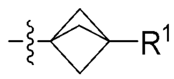 ;

$R^1$ is selected from H, halogen, OH, $NH_2$, SH, CN, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ cycloalkyl, or 4- to 10-membered heterocyclyl, wherein the OH, $NH_2$, SH, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ cycloalkyl, or 4- to 10-membered heterocyclyl is optionally substituted with $R^{1a}$;

$R^{1a}$ is selected from halogen, CN, OH, $NH_2$, or $C_1$-$C_6$ alkyl, wherein the OH, $NH_2$, or $C_1$-$C_6$ alkyl is optionally substituted with $R^{1b}$;

$R^{1b}$ is selected from halogen or $C_1$-$C_6$ alkyl;

$R^2$ is selected from H, D, or $C_1$-$C_6$ alkyl;

$R^3$ and $R^4$ are independently selected from H, D, halogen, OH, $C_1$-$C_6$ alkyl, $C_2$-$C_6$ alkenyl, $C_2$-$C_6$ alkynyl, $C_3$-$C_6$cycloalkyl, or 4- to 7-membered heterocyclyl, wherein the OH, $C_1$-$C_6$ alkyl, $C_2$-$C_6$alkenyl, $C_2$-$C_6$ alkynyl, $C_3$-$C_6$ cycloalkyl, or 4- to 7-membered heterocyclyl is optionally substituted with $R^{3a}$; or $R^3$ and $R^4$, together with the atom to which they are attached, form $C_3$-$C_6$ cycloalkyl or 4- to 7-membered heterocyclyl, wherein the $C_3$-$C_6$ cycloalkyl or 4-to 7-membered heterocyclyl is optionally substituted with $R^{3a}$;

$R^{3a}$ is selected from D, halogen, OH, or $C_1$-$C_6$ alkyl.

In another aspect, the present disclosure provides a pharmaceutical composition, which comprises the compound of formula (I) or the stereoisomer thereof or the pharmaceutically acceptable salt thereof described herein, and optionally a pharmaceutically acceptable excipient.

In yet another aspect, the present disclosure provides the compound of formula (I) or the stereoisomer thereof or the pharmaceutically acceptable salt thereof described herein or the pharmaceutical composition described herein for use in treating or preventing a disease or disorder mediated by QPCT and/or QPCTL in a subject in need thereof.

## DETAILED DESCRIPTION

The present disclosure relates to a compound of formula (A-I) or a stereoisomer thereof or a pharmaceutically acceptable salt thereof,

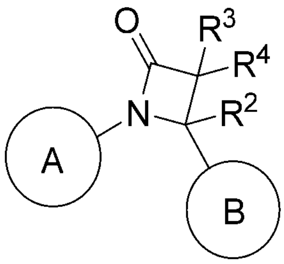

(A-I)

wherein

ring A is selected from

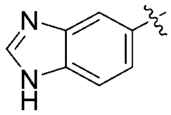

or

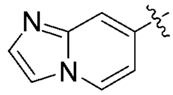

,

wherein the

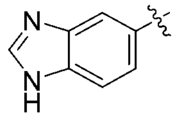

or

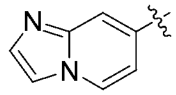

is optionally substituted with $R^5$;

$R^5$ is selected from D, halogen, CN, $NH_2$, OH, $C_1$-$C_6$ alkyl, $C_1$-$C_6$ alkoxy, $C_1$-$C_6$ alkylamino, $C_1$-$C_6$ haloalkyl, or $C_1$-$C_6$ deuterated alkyl;

ring B is selected from

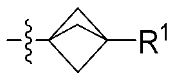

;

$R^1$ is selected from H, halogen, OH, $NH_2$, SH, CN, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ cycloalkyl, or 4- to 10-membered heterocyclyl, wherein the OH, $NH_2$, SH, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ cycloalkyl, or 4- to 10-membered heterocyclyl is optionally substituted with $R^{1a}$; $R^{1a}$ is selected from halogen, CN, OH, $NH_2$, or $C_1$-$C_6$ alkyl, wherein the OH, $NH_2$, or $C_1$-$C_6$ alkyl is optionally substituted with $R^{1b}$;

$R^{1b}$ is selected from halogen or $C_1$-$C_6$ alkyl;

$R^2$ is selected from H, D, or $C_1$-$C_6$ alkyl;

$R^3$ and $R^4$ are independently selected from H, D, halogen, OH, $C_1$-$C_6$ alkyl, $C_2$-$C_6$ alkenyl, $C_2$-$C_6$ alkynyl, $C_3$-$C_6$cycloalkyl, or 4- to 7-membered heterocyclyl, wherein the OH, $C_1$-$C_6$ alkyl, $C_2$-$C_6$alkenyl, $C_2$-$C_6$ alkynyl, $C_3$-$C_6$ cycloalkyl, or 4- to 7-membered heterocyclyl is optionally substituted with $R^{3a}$; or $R^3$ and $R^4$, together with the atom to which they are attached, form $C_3$-$C_6$ cycloalkyl or 4- to 7-membered heterocyclyl, wherein the $C_3$-$C_6$ cycloalkyl or 4- to 7-membered heterocyclyl is optionally substituted with $R^{3a}$;

$R^{3a}$ is selected from D, halogen, OH, or $C_1$-$C_6$ alkyl.

In some embodiments, the compound of formula (A-I) or the stereoisomer thereof or the pharmaceutically

acceptable salt thereof is selected from a compound of formula (I) or a stereoisomer thereof or a pharmaceutically acceptable salt thereof,

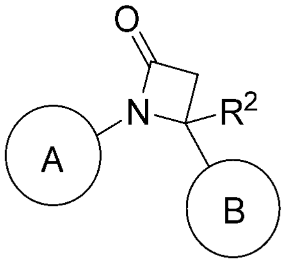

(I)

wherein

ring A is selected from

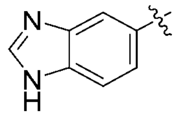 or 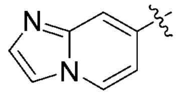 ;

ring B is selected from

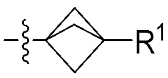 ;

$R^1$ is selected from H, halogen, OH, $NH_2$, SH, CN, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ cycloalkyl, or 4- to 10-membered heterocyclyl, wherein the OH, $NH_2$, SH, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ cycloalkyl, or 4- to 10-membered heterocyclyl is optionally substituted with $R^{1a}$;

$R^{1a}$ is selected from halogen, CN, OH, $NH_2$, or $C_1$-$C_6$ alkyl, wherein the OH, $NH_2$, or $C_1$-$C_6$ alkyl is optionally substituted with $R^{1b}$;

$R^{1b}$ is selected from halogen or $C_1$-$C_6$ alkyl;

$R^2$ is selected from H, D, or $C_1$-$C_6$ alkyl. In some embodiments, ring A is selected from

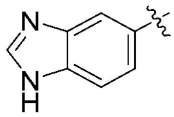 ,

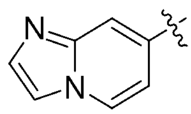 , or 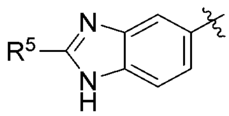 .

In some embodiments, ring A is selected from

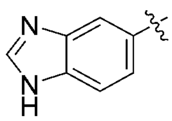 , 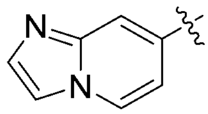 , or 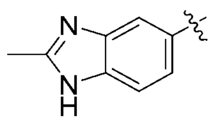 .

In some embodiments, ring A is selected from

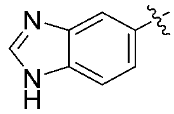 or 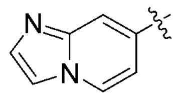 .

In some embodiments, $R^5$ is selected from $C_1$-$C_6$ alkyl.

In some embodiments, $R^5$ is selected from $CH_3$.

In some embodiments, $R^1$ is selected from H, halogen, OH, $NH_2$, $C_1$-$C_6$ alkyl, phenyl, 5- to 6-membered heteroaryl, or 4- to 7-membered heterocyclyl, wherein the OH, $NH_2$, $C_1$-$C_6$ alkyl, phenyl, 5- to 6-membered heteroaryl, or 4- to 7-membered heterocyclyl is optionally substituted with $R^{1a}$.

In some embodiments, $R^1$ is selected from H, halogen, OH, phenyl, 5- to 6-membered heteroaryl, or 4- to 7-membered heterocyclyl, wherein the OH, phenyl, 5- to 6-membered heteroaryl, or 4- to 7-membered heterocyclyl is optionally substituted with $R^{1a}$.

In some embodiments, $R^1$ is selected from H, halogen, OH, $NH_2$, $C_1$-$C_6$ alkyl, phenyl, pyridinyl, or morpholinyl, wherein the OH, $NH_2$, $C_1$-$C_6$ alkyl, phenyl, pyridinyl, or morpholinyl is optionally substituted with $R^{1a}$.

In some embodiments, $R^1$ is selected from H, halogen, OH, phenyl, pyridinyl, or morpholinyl, wherein the OH, phenyl, pyridinyl, or morpholinyl is optionally substituted with $R^{1a}$.

In some embodiments, $R^1$ is selected from H, F, Cl, OH, $NH_2$, $CH_3$,

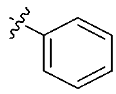 , 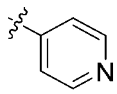 ,

or

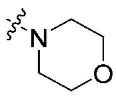 ,

wherein the OH, $NH_2$, $CH_3$,

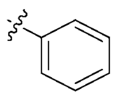 , 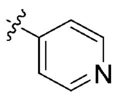 , or 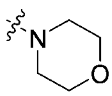

is optionally substituted with $R^{1a}$.

In some embodiments, $R^1$ is selected from H, F, OH,

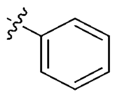 , 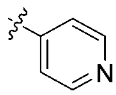 , or 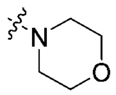 ,

wherein the OH,

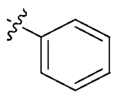 , 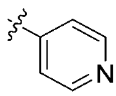 , or 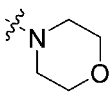

is optionally substituted with $R^{1a}$.

In some embodiments, $R^{1a}$ is selected from halogen, CN, or $C_1$-$C_6$ alkyl.

In some embodiments, $R^{1a}$ is selected from F, CN, $CH_3$, or propyl.

In some embodiments, $R^{1a}$ is selected from F, CN, or propyl.

In some embodiments, $R^1$ is selected from H, F, Cl, $CH_3$, $CH_2F$, $OCH_3$,

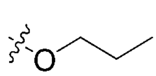, 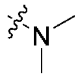,

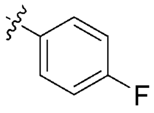, 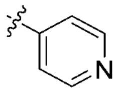, 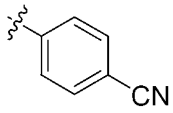, or 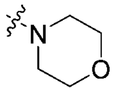.

In some embodiments, $R^1$ is selected from H, F,

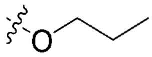, 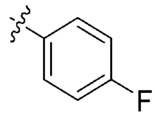, 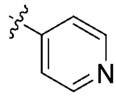,

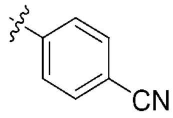, or 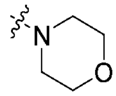.

In some embodiments, $R^2$ is selected from H or $C_1$-$C_6$ alkyl.
In some embodiments, $R^2$ is selected from H or D.
In some embodiments, $R^2$ is selected from H.
In some embodiments, $R^3$ and $R^4$ are independently selected from H, OH, or $C_1$-$C_6$ alkyl, wherein the OH or $C_1$-$C_6$ alkyl is optionally substituted with $R^{3a}$.
In some embodiments, $R^3$ and $R^4$ are independently selected from H, D, OH, $C_1$-$C_6$ alkyl, $C_2$-$C_6$ alkenyl, $C_2$-$C_6$ alkynyl, $C_3$-$C_6$cycloalkyl, or 4- to 7-membered heterocyclyl, wherein the OH, $C_1$-$C_6$alkyl, $C_2$-$C_6$alkenyl, $C_2$-$C_6$ alkynyl, $C_3$-$C_6$ cycloalkyl, or 4- to 7-membered heterocyclyl is optionally substituted with $R^{3a}$; or $R^3$ and $R^4$, together with the atom to which they are attached, form $C_3$-$C_6$ cycloalkyl or 4- to 7-membered heterocyclyl, wherein the $C_3$-$C_6$ cycloalkyl or 4- to 7-membered heterocyclyl is optionally substituted with $R^{3a}$.
In some embodiments, $R^3$ and $R^4$ are independently selected from H, halogen, or $C_1$-$C_6$ alkyl, or $R^3$ and $R^4$, together with the atom to which they are attached, form $C_3$-$C_6$ cycloalkyl, wherein the $C_1$-$C_6$ alkyl and $C_3$-$C_6$ cycloalkyl are optionally substituted with $R^{3a}$.
In some embodiments, $R^3$ and $R^4$ are independently selected from H, halogen, or $C_1$-$C_6$ alkyl, wherein the $C_1$-$C_6$ alkyl is optionally substituted with $R^{3a}$.
In some embodiments, $R^3$ and $R^4$, together with the atom to which they are attached, form $C_3$-$C_6$ cycloalkyl, wherein the $C_3$-$C_6$ cycloalkyl is optionally substituted with $R^{3a}$.
In some embodiments, $R^{3a}$ is selected from halogen or $C_1$-$C_6$ alkyl.
In some embodiments, $R^3$ and $R^4$ are independently selected from H, F, or methyl, or $R^3$ and $R^4$, together with the atom to which they are attached, form cyclobutyl.
In some embodiments, $R^3$ and $R^4$ are both H.
In some embodiments, the compound of formula (A-I) or the stereoisomer thereof or the pharmaceutically acceptable salt thereof of the present disclosure is selected from a compound of formula (II) or a stereoisomer thereof or a pharmaceutically acceptable salt thereof,

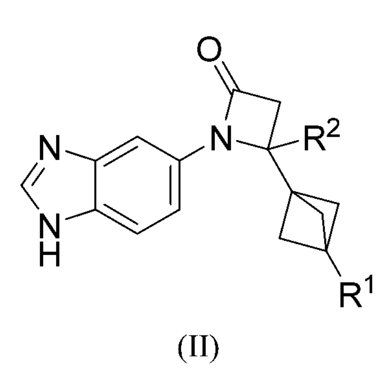

(II)

wherein $R^1$ and $R^2$ are as defined above for formula (A-I). In some embodiments, the compound of formula (A-I) or the stereoisomer thereof or the pharmaceutically acceptable salt thereof of the present disclosure is selected from a compound of formula (II-1) or a stereoisomer thereof or a pharmaceutically acceptable salt thereof,

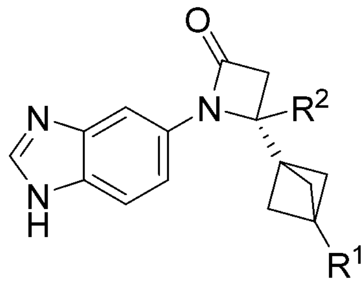

(II-1)

wherein $R^1$ and $R^2$ are as defined above for formula (A-I). In some embodiments, the compound of formula (A-I) or the stereoisomer thereof or the pharmaceutically acceptable salt thereof of the present disclosure is selected from a compound of formula (III) or a stereoisomer thereof or a pharmaceutically acceptable salt thereof,

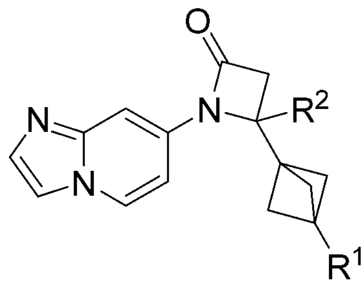

(III)

wherein $R^1$ and $R^2$ are as defined above for formula (A-I).

In some embodiments, the compound of formula (A-I) or the stereoisomer thereof or the pharmaceutically acceptable salt thereof of the present disclosure is selected from a compound of formula (III-1) or a stereoisomer thereof or a pharmaceutically acceptable salt thereof,

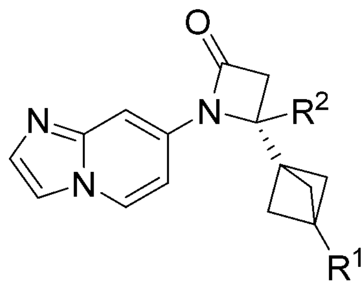

(III-1)

wherein $R^1$ and $R^2$ are as defined above for formula (A-I).

In some embodiments, the compound of formula (A- I) or the stereoisomer thereof or the pharmaceutically acceptable salt thereof of the present disclosure is selected from the following compounds or stereoisomers thereof or pharmaceutically acceptable salts thereof:

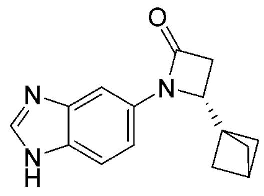

**1**

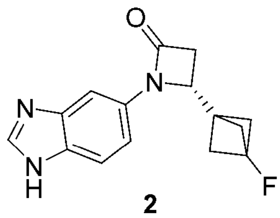

**2**

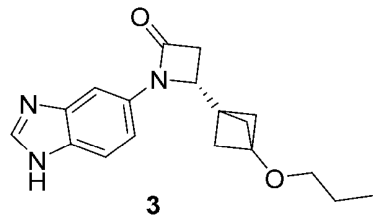

**3**

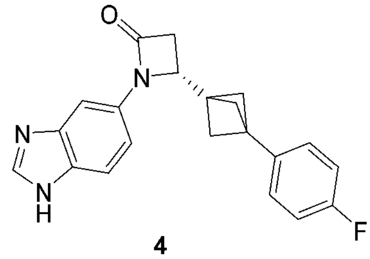

**4**

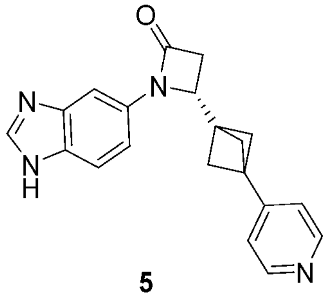

**5**

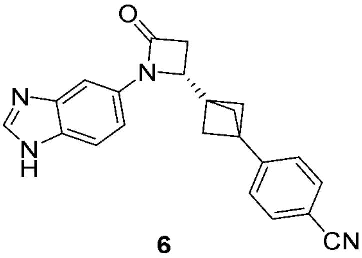

**6**

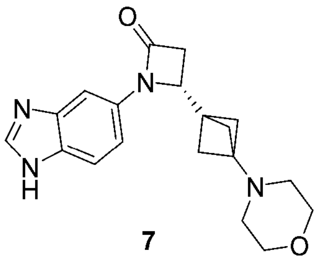

**7**

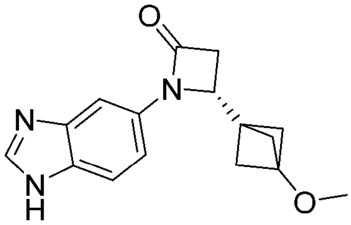

**8**

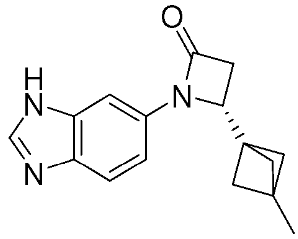

**9**

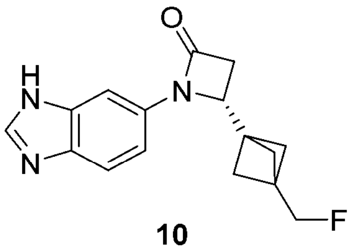

**10**

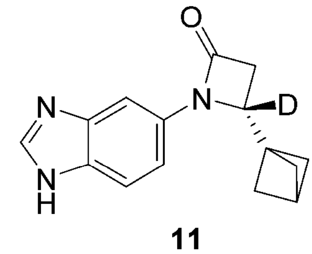

**11**

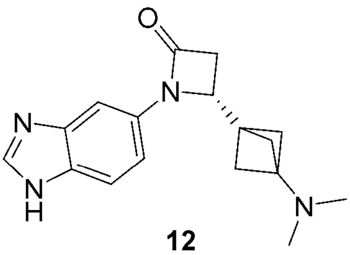

**12**

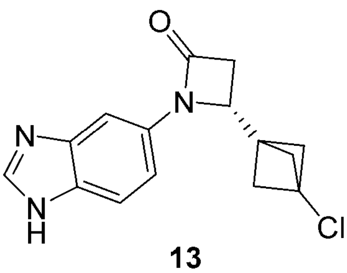

**13**

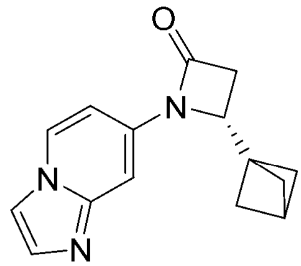

**14**

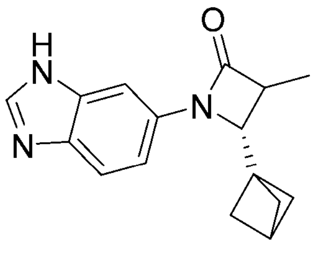

**15**

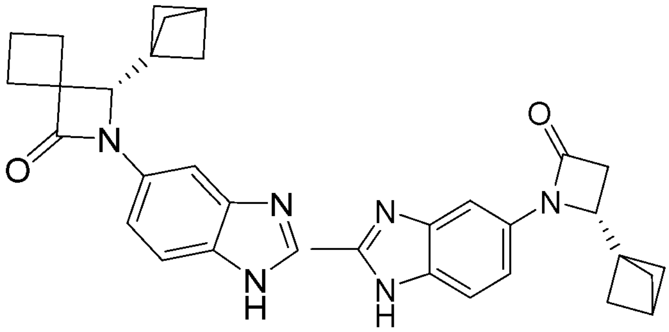

**16** **17** or 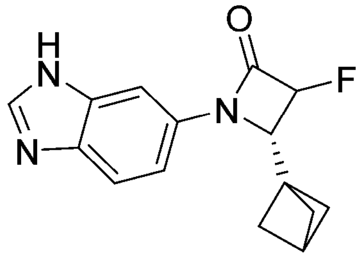 **18** .

In another aspect, the present disclosure provides a pharmaceutical composition, which comprises the compound of formula (A-I) or the stereoisomer thereof or the pharmaceutically acceptable salt thereof of the present disclosure, and a pharmaceutically acceptable excipient.

In another aspect, the present disclosure provides a method for treating a disease mediated by QPCT and/or QPCTL in a mammal, which comprises administering to a mammal, preferably a human, in need of such treatment a therapeutically effective amount of the compound of formula (A-I) or the stereoisomer thereof or the pharmaceutically acceptable salt thereof, or the pharmaceutical composition thereof.

In another aspect, the present disclosure provides use of the compound of formula (A-I) or the stereoisomer thereof or the pharmaceutically acceptable salt thereof, or the pharmaceutical composition thereof in preparing a medicament for preventing or treating a disease mediated by QPCT and/or QPCTL.

In another aspect, the present disclosure provides use of the compound of formula (A-I) or the stereoisomer thereof or the pharmaceutically acceptable salt thereof, or the pharmaceutical composition thereof in preventing or treating a disease mediated by QPCT and/or QPCTL.

In another aspect, the present disclosure provides the compound of formula (A-I) or the stereoisomer thereof or the pharmaceutically acceptable salt thereof, or the pharmaceutical composition thereof for use in preventing or treating a disease mediated by QPCT and/or QPCTL.

In some embodiments, the disease mediated by QPCT and/or QPCTL is selected from a neurodegenerative disease.

In some embodiments, the neurodegenerative disease is Alzheimer's disease.

Terminology and Definitions

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered indefinite or unclear, but should be interpreted according to its common meaning in the field. When referring to a trade name, it is intended to refer to its corresponding commercial product or its active ingredient.

Herein,

“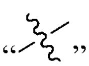”

denotes a linking site.

The illustration method for the racemate or enantiomerically pure compounds herein is from Maehr, J. Chem. Ed. 1985, 62:114-120. Unless otherwise stated, the absolute configuration of a stereogenic center is represented by a wedged bond and a wedged dashed bond (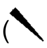 and 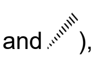), and the relative configuration of a stereogenic center (e.g., cis- or trans-configuration of alicyclic compounds) is represented by a black solid bond and a dashed bond (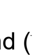 and 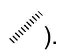).

The term "stereoisomer" refers to isomers resulting from different spatial arrangements of atoms in a molecule, including cis-trans isomers, enantiomers, and diastereoisomers.

The compound of the present disclosure includes tautomeric forms thereof. The tautomer refers to an isomer that results from the rapid movement of an atom between two positions in a molecule. The tautomers may be interconverted

and may coexist in a certain state where an equilibrium state is reached. Examples of tautomers include, but are not limited to,

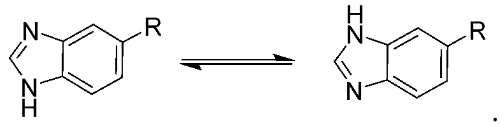

The compound of the present disclosure may have an asymmetric atom such as a carbon atom, a sulfur atom, a nitrogen atom, and a phosphorus atom, or an asymmetric double bond, and thus the compound of the present disclosure may exist in the form of a particular geometric isomer or stereoisomer. The form of a particular geometric isomer or stereoisomer may be cis and trans isomers, E and Z geometric isomers, (-)- and (+)-enantiomers, (R)- and (S)-enantiomers, diastereoisomers, (D)-isomers, (L)-isomers, and racemic mixtures or other mixtures thereof, such as an enantiomer- or diastereoisomer-enriched mixture, and all of the above isomers, as well as mixtures thereof, are encompassed within the definition scope of the compound of the present disclosure. An additional asymmetric carbon atom, asymmetric sulfur atom, asymmetric nitrogen atom, or asymmetric phosphorus atom may be present in substituents such as alkyl. All of these isomers and mixtures thereof involved in the substituents are also encompassed within the definition scope of the compound of the present disclosure. The compound containing an asymmetric atom of the present disclosure can be separated in an optically active pure form or in a racemic form. The optically active pure form can be obtained by resolving a racemic mixture or by synthesis using chiral starting materials or chiral reagents.

The term "substituted" means that any one or more hydrogen atoms on a specific atom are substituted with substituents, as long as the valence of the specific atom is normal and the compound resulting from the substitution is stable. When the substituent is oxo (i.e., =O), it means that two hydrogen atoms are substituted, and oxo will not be present on an aromatic group.

The term "optional" or "optionally" means that the subsequently described event or circumstance may or may not occur. The description includes instances where the event or circumstance occurs and instances where the event or circumstance does not. For example, ethyl being "optionally" substituted with halogen means that the ethyl may be unsubstituted ($CH_2CH_3$), monosubstituted ($CH_2CH_2F$, $CH_2CH_2Cl$, or the like), polysubstituted ($CHFCH_2F$, $CH_2CHF_2$, $CHFCH_2Cl$, $CH_2CHCl_2$, or the like), or fully substituted ($CF_2CF_3$, $CF_2CCl_3$, $CCl_2CCl_3$, or the like). Those skilled in the art will appreciate that for any group containing one or more substituents, no substitution or substituting pattern that is spatially impossible and/or cannot be synthesized will be introduced.

When any variable (e.g., $R^a$ or $R^b$) occurs once or more in the constitution or structure of a compound, the variable is independently defined in each case. For example, if a group is substituted with 2 $R^b$, the definition of each $R^b$ is independent.

$C_m$-$C_n$ used herein means having an integer number of carbon atoms in the range of m-n. For example, the "$C_1$-$C_{10}$" means that the group may have 1 carbon atom, 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms.

The term "alkyl" refers to a hydrocarbyl group with a general formula of $C_nH_{2n+1}$. The alkyl may be linear or branched. The term "$C_1$-$C_6$ alkyl" should be understood to represent a linear or branched saturated monovalent hydrocarbyl group having 1, 2, 3, 4, 5, or 6 carbon atoms. Specific examples of the alkyl include, but are not limited to, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like. The term "$C_1$-$C_3$ alkyl" may be understood to represent a linear or branched saturated monovalent hydrocarbyl group having 1 to 3 carbon atoms. The "$C_1$-$C_{10}$ alkyl" may include the ranges of "$C_1$-$C_6$ alkyl", "$C_1$-$C_3$ alkyl", and the like, and the "$C_1$-$C_6$ alkyl" may further include "$C_1$-$C_3$ alkyl".

The term "alkenyl" refers to a linear or branched unsaturated aliphatic hydrocarbyl group consisting of carbon atoms and hydrogen atoms and having at least one double bond. The term "$C_2$-$C_6$ alkenyl" should be understood to represent a linear or branched unsaturated monovalent hydrocarbyl group comprising one or more double bonds and having 2, 3, 4, 5, or 6 carbon atoms, and the "$C_2$-$C_6$ alkenyl" is preferably "$C_2$-$C_4$ alkenyl", further preferably $C_2$ alkenyl or $C_3$ alkenyl. It should be understood that in the case that the alkenyl comprises more than one double bond, the double bonds can be separated from one another or conjugated. Specific examples of the alkenyl include, but are not limited to, vinyl, allyl, (E)-2-methylvinyl, (Z)-2-methylvinyl, (E)-but-2-enyl, (Z)-but-2-enyl, (E)-but-1-enyl, (Z)-but-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, and the like.

The term "alkynyl" refers to a linear or branched unsaturated aliphatic hydrocarbyl group consisting of carbon atoms and hydrogen atoms and having at least one triple bond. The term "$C_2$-$C_6$ alkynyl" may be understood to represent a linear or branched unsaturated monovalent hydrocarbyl group comprising one or more triple bonds and having 2, 3, 4, 5, or 6 carbon atoms. Examples of the "$C_2$-$C_6$ alkynyl" include, but are not limited to, ethynyl (-C≡CH), propynyl (-C≡$CCH_3$ or -$CH_2$C≡CH), but-1-ynyl, but-2-ynyl, and but-3-ynyl. The "$C_2$-$C_6$ alkynyl" may include "$C_2$-$C_3$ alkynyl", and examples of the

"$C_2$-$C_3$ alkynyl" include ethynyl (-C≡CH), prop-1-ynyl (-C≡$CCH_3$), and prop-2-ynyl (propargyl).

The term "cycloalkyl" refers to a fully saturated carbon ring that exists in the form of a monocyclic ring, a fused ring, a bridged ring, a spiro ring, or the like. The term "$C_3$-$C_6$ cycloalkyl" may be understood to represent a saturated monovalent monocyclic or bicyclic hydrocarbyl ring having 3, 4, 5, or 6 carbon atoms. Specific examples include, but are not limited to, cyclopropyl, cyclobutyl, cyclopentyl, cyclohexyl, or the like.

The term "aryl" refers to an aromatic all-carbon monocyclic or fused polycyclic group with a conjugated $\pi$-electron system. The term "$C_6$-$C_{10}$ aryl" should be understood as an aromatic or partially aromatic monovalent all-carbon monocyclic or bicyclic group having 6-10 carbon atoms, particularly a ring having 6 carbon atoms ("$C_6$ aryl"), such as phenyl, or a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl.

The term "heteroaryl" refers to an aromatic cyclic group having an aromatic monocyclic or fused polycyclic system, which contains at least one ring atom selected from N, O, and S, with the remaining ring atoms being C. The term "5- to 10-membered heteroaryl" should be understood to include a monovalent aromatic monocyclic or bicyclic ring system, which has 5, 6, 7, 8, 9, or 10 ring atoms, particularly 5, 6, 9, or 10 ring atoms, and comprises 1, 2, 3, 4, or 5, preferably 1, 2, or 3, heteroatoms independently selected from N, O, and S. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, and the like, and benzo derivatives thereof, such as benzofuranyl, benzothienyl, benzothiazolyl, benzoxazolyl, benzoisoxazolyl, benzimidazolyl, benzotriazolyl, indazolyl, indolyl, isoindolyl, and the like; or pyridyl, pyridazinyl, pyrimidinyl, pyrazinyl, triazinyl, and the like, and benzo derivatives thereof, such as quinolyl, quinazolinyl, isoquinolyl, and the like; or azocinyl, indolizinyl, purinyl, and the like, and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like. The term "5- to 6-membered heteroaryl" refers to an aromatic ring system that has 5 or 6 ring atoms and comprises 1-3, preferably 1-2, heteroatoms independently selected from N, O, and S.

The term "heterocyclyl" refers to a fully saturated or partially saturated (non-aromatic heteroaromatic group on the whole) monovalent monocyclic, fused cyclic, spiro cyclic, or bridged cyclic group, and ring atoms of the group include 12, 3, 4, or 5 heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), $-S(=O)_2-$, -S(=O)-, and optionally substituted -NH-, -S(=O)(=NH)-, -C(=O)NH-, -C(=NH)-, -$S(=O)_2NH-$, S(=O)NH-, -NHC(=O)NH-, and the like. The term "4- to 10-membered heterocyclyl" refers to a heterocyclyl group having 4, 5, 6, 7, 8, 9, or 10 ring atoms, and 1-5 ring atoms of the group are independently selected from the heteroatoms and heteroatom groups described above. "4- to 10-membered heterocyclyl" includes "4- to 7-membered heterocyclyl", wherein specific examples of 4-membered heterocyclyl include, but are not limited to, azetidinyl and oxetanyl; specific examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazolyl, and 2,5-dihydro-1H-pyrrolyl; specific examples of 6-membered heterocyclyl include, but are not limited to, tetrahydropyranyl, piperidyl, morpholinyl, dithianyl, thiomorpholinyl, piperazinyl, trithianyl, tetrahydropyridinyl, and 4H-[1,3,4]thiadiazinyl; specific examples of 7-membered heterocyclyl include, but are not limited to, diazepanyl. The heterocyclyl may also be a bicyclic group, wherein specific examples of 5,5-membered bicyclic groups include, but are not limited to, hexahydrocyclopenta[c]pyrrol-2(1H)-yl; specific examples of 5,6-membered bicyclic groups include, but are not limited to, hexahydropyrrolo[1,2-a]pyrazin-2(1H)-yl, 5,6,7,8-tetrahydro-[1,2,4]triazolo[4,3-a]pyrazinyl, and 5,6,7,8-tetrahydroimidazo[1,5-a]pyrazinyl. Optionally, the heterocyclyl may be a benzo-fused ring group of the 4- to 7-membered heterocyclyl described above. Specific examples include, but are not limited to, dihydroisoquinolyl and the like. "4- to 10-membered heterocyclyl" may include the ranges of "5- to 10-membered heterocyclyl", "4- to 7-membered heterocyclyl", "5- to 6-membered heterocyclyl", "6- to 8-membered heterocyclyl", "4- to 10-membered heterocycloalkyl", "5- to 10-membered heterocycloalkyl", "4- to 7-membered heterocycloalkyl", "5- to 6-membered heterocycloalkyl", "6- to 8-membered heterocycloalkyl", and the like, and the "4- to 7-membered heterocyclyl" may further include the ranges of "4- to 6-membered heterocyclyl", "5- to 6-membered heterocyclyl", "4- to 7-membered heterocycloalkyl", "4- to 6-membered heterocycloalkyl", "5- to 6-membered heterocycloalkyl", and the like. Although some bicyclic heterocyclyl groups herein comprise, in part, one benzene ring or one heteroaromatic ring, the heterocyclyl is still non-aromatic on the whole.

The term "halo" or "halogen" refers to fluorine, chlorine, bromine, or iodine.

The term "hydroxyl" refers to the -OH group.

The term "cyano" refers to the -CN group.

The term "amino" refers to the $-NH_2$ group.

The term "alkoxy" refers to a linear or branched alcohol in which a hydrogen atom on the hydroxyl group is substituted with alkyl. The term "$C_1$-$C_6$ alkoxy" may further include "$C_1$-$C_3$ alkoxy".

The term "alkylamino" refers to an amino group in which one or two hydrogen atoms are substituted with identical or different alkyl groups. The term "$C_1$-$C_6$ alkylamino" may further include "$C_1$-$C_3$ alkylamino".

The term "haloalkyl" refers to an alkyl group in which one or more hydrogen atoms are substituted with identical or

different halogen atoms. The term "$C_1$-$C_6$ haloalkyl" may further include "$C_1$-$C_3$ haloalkyl".

The term "deuterated alkyl" refers to an alkyl group in which one or more hydrogen atoms are substituted with deuterium atoms. The term "$C_1$-$C_6$ deuterated alkyl" may further include "$C_1$-$C_3$ deuterated alkyl".

The term "treating" or "treatment" means administering the compound or formulation described herein to ameliorate or eliminate a disease or one or more symptoms associated with the disease, and includes:

(i) inhibiting a disease or a disease state, i.e., arresting its progression; and
(ii) alleviating a disease or a disease state, i.e., causing its regression.

The term "preventing" or "prevention" means administering the compound or formulation described herein to prevent a disease or one or more symptoms associated with the disease, and includes: preventing the development of the disease or disease state in a mammal, particularly when such a mammal is predisposed to the disease state but has not yet been diagnosed with it.

The term "therapeutically effective amount" refers to an amount of the compound of the present disclosure for (i) treating a specific disease, condition, or disorder; (ii) alleviating, ameliorating, or eliminating one or more symptoms of a specific disease, condition, or disorder; or (iii) delaying the onset of the one or more symptoms of the specific disease, condition, or disorder described herein. The amount of the compound of the present disclosure constituting the "therapeutically effective amount" varies depending on the compound, the disease state and its severity, the mode of administration, and the age of the mammal to be treated, but can be determined conventionally by those skilled in the art in accordance with their knowledge and the content of the present disclosure. The term "patient" includes mammals and non-mammals. Examples of mammals include, but are not limited to, any member of the class Mammalia: humans, non-human primates (e.g., chimpanzees and other apes and monkeys); livestock animals, such as cattle, horses, sheep, goats, and pigs; domestic animals, such as rabbits, dogs, and cats; laboratory animals, including rodents, such as rats, mice, and guinea pigs. Examples of non-human mammals include, but are not limited to, birds, fish, and the like.

The term "a disease or disorder mediated by QPCT and/or QPCTL" includes, but is not limited to, neurodegenerative diseases, cancer, and the like. The neurodegenerative diseases include Alzheimer's disease (AD), Parkinson's disease (PD), Huntington's disease (HD), amyotrophic lateral sclerosis (ALS), different types of spinocerebellar ataxia (SCA), Pick's disease, and the like.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to salts of pharmaceutically acceptable acids or bases, including salts formed from the compound and an inorganic or organic acid, and salts formed from the compound and an inorganic or organic base.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, water, and the like.

The word "comprise" and variations thereof such as "comprises" or "comprising" should be understood in an open and non-exclusive sense, i.e., "including but not limited to".

The present disclosure also includes isotopically labeled compounds of the present disclosure that are identical to those documented herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Certain isotopically labeled compounds of the present disclosure (e.g., those labeled with $^{3}H$ and $^{14}C$) can be used to analyze compounds and/or substrate tissue distribution. Tritium (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$, and $^{18}F$, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

The pharmaceutical composition of the present disclosure can be prepared by combining the compound of the present disclosure with a suitable pharmaceutically acceptable excipient, and can be formulated, for example, into a solid,

semisolid, liquid, or gaseous formulation such as tablet, pill, capsule, powder, granule, ointment, emulsion, suspension, suppository, injection, inhalant, gel, microsphere, aerosol, and the like.

Typical routes of administration of the compound or the pharmaceutically acceptable salt thereof or the pharmaceutical composition thereof of the present disclosure include, but are not limited to, oral, rectal, local, inhalation, parenteral, sublingual, intravaginal, intranasal, intraocular, intraperitoneal, intramuscular, subcutaneous, and intravenous administration.

The pharmaceutical composition of the present disclosure can be manufactured by methods well known in the art, such as conventional methods of mixing, dissolving, granulating, emulsifying, freeze-drying, and the like.

In some embodiments, the pharmaceutical composition is in an oral form. For oral administration, the pharmaceutical composition can be formulated by mixing the active compound with pharmaceutically acceptable excipients well known in the art. These excipients enable the compounds of the present disclosure to be formulated into tablets, pills, lozenges, dragees, capsules, liquids, gels, slurries, suspensions, and the like, for oral administration to patients.

A solid oral composition can be prepared by conventional mixing, filling, or tableting. For example, it can be obtained by the following method: mixing the active compound with a solid excipient, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, flavoring agents, and the like.

The pharmaceutical composition may also be suitable for parenteral administration, such as a sterile solution, suspension, or lyophilized product in a suitable unit dosage form.

In all of the administration methods of the compound of general formula I described herein, the daily dose is from 0.01 mg/kg body weight to 1000 mg/kg body weight, e.g., from 0.01 mg/kg body weight to 500 mg/kg body weight, given in a single dose or divided doses.

The compounds of the present disclosure can be prepared by a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions of the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction process based on the existing embodiments.

The following abbreviations are used in the present disclosure:

$Ti(OiPr)_4$: tetraisopropyl titanate; THF: tetrahydrofuran; MeOH: methanol; LDA: lithium diisopropylamide; CuI: cuprous iodide; dioxane: dioxane; TFA: trifluoroacetic acid; DCM: dichloromethane; EBA: ethyl bromoacetate; TMSCl: trimethylsilyl chloride; SEM: (trimethylsilyl)ethoxymethyl; TsOH: *p*-toluenesulfonic acid; Pd/C: palladium/carbon; LAH or $LiAlH_4$: lithium aluminum hydride; DMP: Dess-Martin periodinane, (1,1,1-triacetoxy)-1,1-dihydro-1,2-benziodoxol-3(1H)-one; DIC: N,N'-diisopropylcarbodiimide; DMAP: 4-dimethylaminopyridine; $NiBr_2$(dme): nickel(II) bromide ethylene glycol dimethyl ether complex; DMA: dimethylacetamide; DIBAL-H: diisobutylaluminum hydride; DMSO: dimethyl sulfoxide; TEA: triethylamine; TBAF: tetrabutylammonium fluoride; $BH_3THF$: solution of borane in tetrahydrofuran; TosCl: p-toluenesulfonyl chloride; DAST: diethylaminosulfur trifluoride; $AlD_4Li$: deuterated lithium aluminum hydride; Oxalyl chloride: oxalyl chloride; Sodium Pyrithione: sodium pyrithione; AIBN: azobisisobutyronitrile; NFSI: *N*-fluorobenzenesulfonimide.

## EXAMPLES

The present disclosure is described in detail below by way of examples, which, however, are not intended to disadvantageously limit the scope of the present disclosure in any way. Although the present disclosure has been described in detail herein and specific embodiments thereof have also been disclosed, it will be apparent to those skilled in the art that various changes and modifications can be made to the specific embodiments of the present disclosure without departing from the spirit and scope of the present disclosure. All reagents used in the present disclosure are commercially available and can be used without further purification.

Unless otherwise stated, the ratios expressed for mixed solvents are volume mixing ratios. Unless otherwise stated, % refers to wt%.

Compounds are named either manually or by ChemDraw® software, and supplier's catalog names are given for commercially available compounds.

The structures of the compounds are determined by nuclear magnetic resonance (NMR) and/or mass spectrometry (MS). NMR shifts are given in $10^{-6}$ (ppm). The solvents for NMR determination are deuterated dimethyl sulfoxide, deuterated chloroform, deuterated methanol, and the like, and the internal standard is tetramethylsilane (TMS); "$IC_{50}$" refers to the half maximal inhibitory concentration, which is the concentration at which half of the maximal inhibitory effect is

achieved. The eluent below may be a mixed eluent formed by two or more solvents, and the ratio is the volume ratio of the solvents. For example, "0-10% ethyl acetate/petroleum ether" means that during gradient elution, the volume ratio of ethyl acetate to petroleum ether in the mixed eluent is 0:100 to 10:90, or "dichloromethane/methanol = 20:1" means that during gradient elution, the volume ratio of dichloromethane to methanol in the mixed eluent is 20:1.

**Preparation of intermediates**

**Synthesis of intermediate 3-1**:

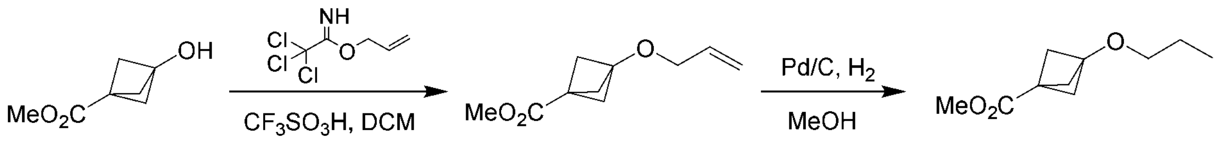


**3-001** **3-002** **3-003**

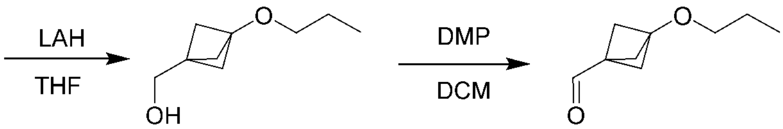


**3-004** **3-1**

**Step 1: synthesis of intermediate 3-002**

**3-001** (3.70 g) was dissolved in dichloromethane (100 mL), and allyl 2,2,2-trichloroacetamide (7.90 g) and trifluoromethanesulfonic acid (781 mg) were added. The mixture was stirred at 25 ° C for 16 h. After the reaction was completed, dichloromethane (200 mL) was added to the reaction mixture for extraction, followed by washing with saturated sodium bicarbonate (50 mL × 2). The organic phase was dried and concentrated to give a crude product. The crude product was purified by silica gel column chromatography (ethyl acetate/petroleum ether = 0%-5%) to give intermediate **3-002** (3.60 g).

$^1$H-NMR(400 MHz, $CDCl_3$) $\delta$ 5.98-5.90 (m, 1H), 5.34-5.29 (m, 1H), 5.22-5.18 (m, 1H), 4.03-4.01 (m, 2H), 3.72 (s, 3H), 2.23 (s, 6H).

**Step 2: synthesis of intermediate 3-003**

**3-002** (3.60 g) was dissolved in methanol (50 mL), and 10% Pd/C (700 mg) was added. The reaction mixture was stirred at 25 °C for 16 h in a hydrogen atmosphere (15 psi). After the reaction was completed, the reaction mixture was filtered, and the filtrate was concentrated under reduced pressure to give a crude product. The crude product was purified by silica gel column chromatography (ethyl acetate/petroleum ether = 0%-5%) to give intermediate **3-003** (2.30 g).

$^1$H-NMR(400 MHz, $CDCl_3$) $\delta$ 3.62 (s, 3H), 3.33 (t, *J* = 6.8 Hz, 2H), 2.12 (s, 6H), 1.58-1.46 (m, 2H), 0.86 (t, *J* = 7.4 Hz, 3H).

**Step 3: synthesis of intermediate 3-004**

**3-003** (2.30 g) was dissolved in tetrahydrofuran (40 mL) in a nitrogen atmosphere, and the solution was cooled to 0 °C. A lithium aluminum hydride solution (2.5 M, 7.49 mL) was slowly added dropwise, the mixture was stirred at 25 °C for 2 h, and then the reaction was completed. Water (8 mL), a 15% sodium hydroxide solution (8 mL), and water (24 mL) were added to the reaction mixture successively to quench the reaction, and the mixture was dried over anhydrous magnesium sulfate, filtered, and concentrated to give crude intermediate **3-004** (1.80 g).

**Step 4: synthesis of intermediate 3-1**

**3-004** (1.80 g) was dissolved in dichloromethane (80 mL) in a nitrogen atmosphere, and the solution was cooled to 0 °C. Dess-Martin periodinane (7.33 g) was added in portions, the mixture was stirred at 0 °C for 2 h, and then the reaction was completed. The reaction mixture was filtered, and the filtrate was diluted with dichloromethane (100 mL), washed

twice with saturated sodium bicarbonate (50 mL), and then washed with saturated brine (50 mL). The organic phase was dried and concentrated to give a crude product. The crude product was purified by silica gel column chromatography (tetrahydrofuran/petroleum ether = 0%-5%) to give intermediate **3-1** (1.10 g).

$^1$H-NMR(400 MHz, $CDCl_3$) $\delta$ 9.70 (s, 1H), 3.47-3.38 (m, 2H), 2.19 (s, 6H), 1.65-1.58 (m, 2H), 0.98-0.91 (m, 3H).

**Synthesis of intermediate 4-1**:

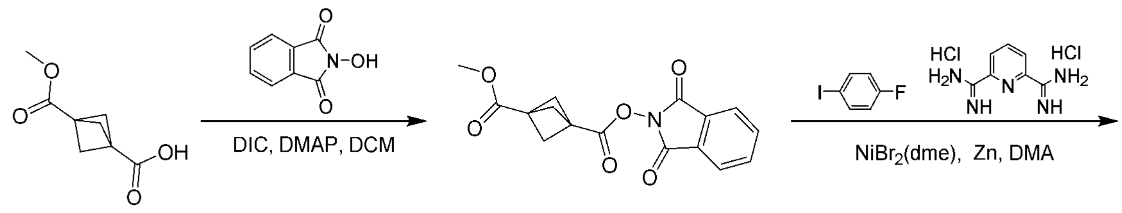


4-001 4-002

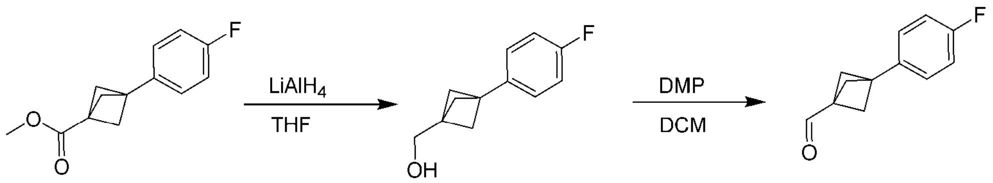


4-003 4-004 4-1

**Step 1: synthesis of intermediate 4-002**

**4-001** (15.0 g) and N-hydroxyphthalimide (14.4 g) were dissolved in dichloromethane (500 mL). 4-Dimethylaminopyridine (1.08 g) and N,N'-diisopropylcarbodiimide (13.4 g) were added at 0 °C. The mixture was stirred at 25 °C for 16 h, and then the reaction was completed. The reaction mixture was filtered, and the filtrate was concentrated to give a crude product. The crude product was purified by silica gel column chromatography (dichloromethane/petroleum ether, gradient 0-100%) to give intermediate **4-002** (14.4 g).

$^1$H-NMR(400 MHz, $CDCl_3$) $\delta$ 7.98-7.88 (m, 2H), 7.86-7.77 (m, 2H), 3.74 (s, 3H), 2.57 (s, 6H).

**Step 2: synthesis of intermediate 4-003**

**4-002** (8.00 g) and p-fluoroiodobenzene (7.89 g) were dissolved in dimethylacetamide (80 mL), and zinc powder (4.31 g), nickel(II) bromide ethylene glycol dimethyl ether complex (783 mg), and pyridine-2,6-bis(carboximidamide) dihydrochloride (599 mg) were added. The reaction mixture was stirred at 25 °C for 16 h in a nitrogen atmosphere, and then the reaction was completed. Ethyl acetate (600 mL) was added for dilution, and the mixture was washed with saturated brine (100 mL × 4). The organic phase was dried over anhydrous sodium sulfate, filtered, and concentrated. The crude product was purified by silica gel column chromatography (gradient: 0-5% ethyl acetate/petroleum ether) to give intermediate **4-003** (1.30 g).

$^1$H-NMR(400 MHz, $CDCl_3$) $\delta$ 7.24-7.11 (m, 2H), 7.08-6.93 (m, 2H), 3.74 (s, 3H), 2.33 (s, 6H).

**Step 3: synthesis of intermediate 4-004**

**4-003** (2.20 g) was dissolved in tetrahydrofuran (25 mL) in a nitrogen atmosphere, and a solution of lithium aluminum hydride in tetrahydrofuran (2.5 M, 5.99 mL) was slowly added dropwise at 0 °C. The mixture was stirred at 25 °C for 16 h, and then the reaction was completed. Water (600 μL), a 15% aqueous sodium hydroxide solution (600 μL), and water (1800 μL) were added successively to the reaction mixture at 0 ° C to quench the reaction, and then anhydrous magnesium sulfate was added. The mixture was stirred for 30 min and then filtered, and the filtrate was concentrated to give intermediate **4-004** (1.80 g).

$^1$H-NMR(400 MHz, $CDCl_3$) $\delta$ 7.26-7.15 (m, 2H), 7.06-6.95 (m, 2H), 3.78-3.68 (m, 2H), 2.00 (s, 6H).

**Step 4: synthesis of intermediate 4-1**

**4-004** (1.80 g) was dissolved in dichloromethane (30 mL) in a nitrogen atmosphere, and Dess-Martin periodinane (5.96 g) was added at 0 °C. The reaction mixture was stirred at 0 °C for 2 h, and then the reaction was completed. Dichloromethane (100 mL) was added to the reaction mixture, and the mixture was washed with a saturated aqueous sodium bicarbonate solution (50 mL × 3). The organic phase was dried and concentrated to give a crude product. The crude product was purified by silica gel column chromatography (gradient: 0-5% ethyl acetate/petroleum ether) to give intermediate **4-1** (1.78 g).
$^1$H-NMR(400 MHz, $CDCl_3$) $\delta$ 9.70 (s, 1H), 7.25-7.14 (m, 2H), 7.08-6.97 (m, 2H), 2.40-2.26 (m, 6H).

**Synthesis of intermediate 5-1 and intermediate 6-1**:

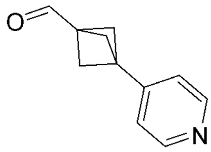

**5-1**

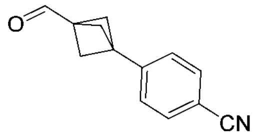

**6-1**

For synthesis of intermediate **5-1** and intermediate **6-1**, reference was made to synthesis procedure of intermediate **4-1**, except that p-fluoroiodobenzene in step **2** was replaced by 4-iodopyridine and 4-iodobenzonitrile, respectively.

**Synthesis of intermediate 7-1:**

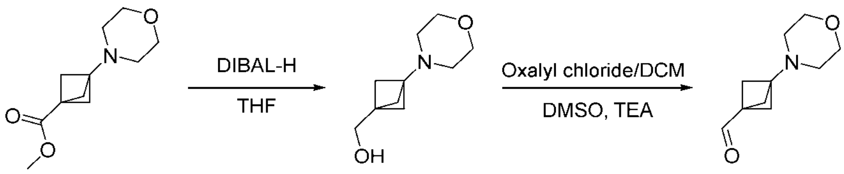


**7-001** **7-002** **7-1**

**Step 1: synthesis of intermediate 7-002:**

**7-001** (3.00 g) was dissolved in tetrahydrofuran (30 mL). The mixture was cooled to -78 °C in a nitrogen atmosphere, and then a solution of DIBAL-H in toluene (1 M, 21.3 mL) was slowly added dropwise over 15 min. The reaction mixture was stirred at -78 °C for 2 h, and then the reaction was completed. The reaction mixture was warmed to 0 °C, and a saturated aqueous ammonium chloride solution was slowly added dropwise to quench the reaction, followed by extraction with ethyl acetate (30.0 mL × 3). The organic phases were combined, dried over anhydrous sodium sulfate, filtered, and concentrated. The crude product was purified by column chromatography (dichloromethane/methanol = 20:1) to give intermediate **7-002** (1.70 g).
$^1$H-NMR (400MHz, DMSO-$d_6$) $\delta$ 4.47 (t, $J$ = 5.6 Hz, 1H), 3.60-3.52 (m, 4H), 3.46 (d, $J$ = 5.6 Hz, 2H), 2.35-2.28 (m, 4H), 1.53 (s, 6H).
MS m/z (ESI) = 184.20

**Step 2: synthesis of intermediate 7-1:**

Dimethyl sulfoxide (1.62 g) was slowly added dropwise to a solution of oxalyl chloride (1.58 g) in dichloromethane (30 mL) at -78 °C in a nitrogen atmosphere. The reaction mixture was stirred at - 78 °C for 1 h. Then, **7-002** (1.70 g) was added to the reaction mixture, and the reaction mixture was stirred at -78 °C for 1 h. Triethylamine (5.25 g) was slowly added to the reaction mixture. The reaction mixture was stirred at -78 °C for 30 min, and then the reaction was completed. The reaction mixture was warmed to 0 °C, water was slowly added dropwise to quench the reaction, and ethyl acetate (30.0 mL × 3) was added for extraction. The organic phases were combined, dried over anhydrous sodium sulfate, filtered, and

concentrated. The crude product was purified by column chromatography (dichloromethane/methanol = 20/1) to give intermediate **7-1** (1.15 g).

$^1$H-NMR (400MHz, DMSO-$d_6$) $\delta$ 9.64 (s, 1H), 3.59-3.57 (m, 4H), 2.39-2.33 (m, 4H), 1.93 (s, 6H). MS m/z (ESI) = 182.1.

**Synthesis of intermediate 9-1**:

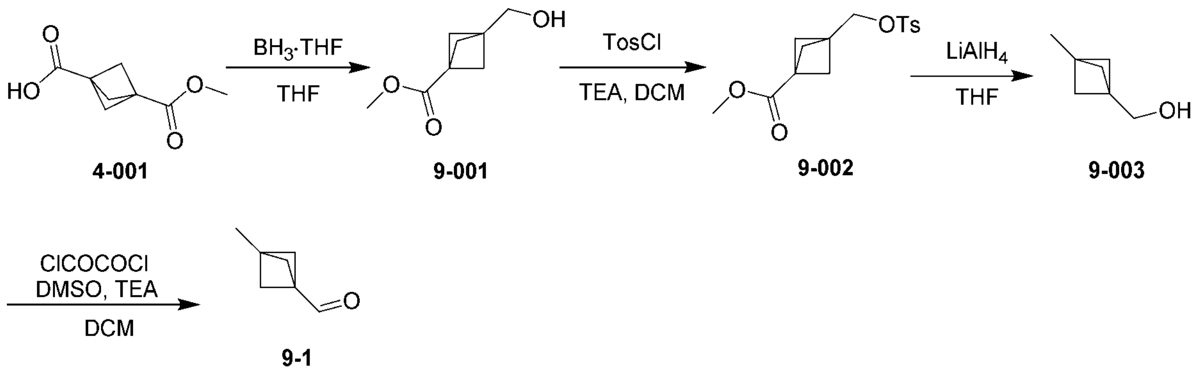


**4-001** **9-001** **9-002** **9-003** **9-1**

**Step 1: synthesis of intermediate 9-001**

**4-001** (5.00 g) was dissolved in tetrahydrofuran (80 mL), and a solution of borane in tetrahydrofuran (1 M, 35.26 mL) was slowly added dropwise at 0 °C in a nitrogen atmosphere. The reaction mixture was stirred at 25 °C for 16 h, and then the reaction was completed. Methanol (80 mL) was added to the reaction mixture at 0 °C, and the mixture was stirred for 30 min. The organic phase was dried and concentrated to give a crude product. The crude product was purified by flash silica gel column chromatography (gradient: 0-20% ethyl acetate/petroleum ether) to give intermediate **9-001** (4.53 g). $^1$H NMR (400 MHz, Chloroform-*d*) $\delta$ 3.61 (s, 3H), 3.56 (s, 2H), 1.93 (s, 6H).

**Step 2: synthesis of intermediate 9-002**

**9-001** (9.50 g), p-toluenesulfonyl chloride (13.9 g), and triethylamine (7.39 g) were dissolved in dichloromethane (100 mL). The reaction mixture was stirred at 25 °C for 16 h, and then the reaction was completed. The reaction mixture was concentrated and purified by silica gel column chromatography (gradient: 0-20% ethyl acetate/petroleum ether) to give intermediate **9-002** (15.6 g). $^1$H-NMR (400 MHz, Chloroform-*d*) $\delta$ 7.79 (d, *J* = 8.1 Hz, 2H), 7.36 (d, *J* = 7.9 Hz, 2H), 4.04 (s, 2H), 3.67 (s, 3H), 2.47 (s, 3H), 1.99 (s, 6H)
MS m/z (ESI): = 311.0 $[M+H]^+$

**Step 3: synthesis of intermediate 9-003**

**9-002** (15.0 g) was dissolved in tetrahydrofuran (200 mL) in a nitrogen atmosphere, and a solution of lithium aluminum hydride in tetrahydrofuran (2.5 M, 38.66 mL) was slowly added dropwise at 0 °C. The reaction mixture was stirred at 25 °C for 16 h, and then the reaction was completed. Water (3.7 mL), a 15% aqueous sodium hydroxide solution (3.7 mL), and water (11.1 mL) were added to the reaction mixture successively, and the mixture was stirred for 10 min, dried over anhydrous magnesium sulfate, and filtered. The filtrate was concentrated to give a crude product. The crude product was purified by silica gel column chromatography (gradient: 0-10% ethyl acetate/petroleum ether) to give intermediate **9-003** (3.14 g).

$^1$H-NMR (400 MHz, Chloroform-*d*) $\delta$ 3.58 (s, 2H), 1.59 (s, 6H), 1.19 (s, 3H).

**Step 4: synthesis of intermediate 9-1**

Oxalyl chloride (4.41 g) was dissolved in dichloromethane (15 mL) in a nitrogen atmosphere, and a solution of dimethyl sulfoxide (5.43 g) in dichloromethane (5 mL) was slowly added dropwise at - 78 °C. The mixture was stirred at -78 °C for 30 min. Then, a solution of **9-003** (2.60 g) in dichloromethane (5 mL) was slowly added dropwise, and the mixture was stirred at -78 °C for 30 min. Finally, a solution of triethylamine (14.1 g) in dichloromethane (20 mL) was slowly added dropwise, the reaction mixture was stirred at 25 °C for 16 h, and then the reaction was completed. The reaction mixture was

washed once with saturated brine (50 mL) and concentrated to 50 mL to give intermediate **9-1**, which was directly used in the next step.

**Synthesis of intermediate 10-1:**

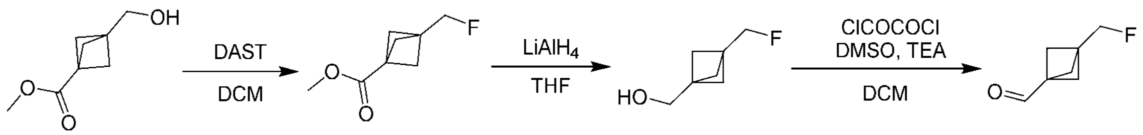


**9-001** **10-001** **10-002** **10-1**

**Step 1: synthesis of intermediate 10-001:**

**9-001** (9.00 g) was dissolved in dichloromethane (100 mL) in a nitrogen atmosphere, and diethylaminosulfur trifluoride (18.5 g) was slowly added dropwise to the reaction mixture at 0 °C. The reaction mixture was stirred at 25 °C for 16 h, and then the reaction was completed. The reaction mixture was slowly poured into a saturated sodium bicarbonate solution (100 mL) with crushed ice under stirring. After stirring, the solution was extracted with dichloromethane (20 mL × 3) and washed with saturated brine (20 mL × 3). The organic phases were combined, dried, and concentrated to give a crude product. The crude product was purified by silica gel column chromatography (gradient: 0-3% tetrahydrofuran/petroleum ether) to give intermediate **10-001** (9.05 g).

$^1$H NMR (400 MHz, Chloroform-*d*) $\delta$ 4.46 (s, 1H), 4.34 (s, 1H), 3.70 (s, 3H), 2.09 (s, 6H)

**Step 2: synthesis of intermediate 10-002:**

**10-001** (5.00 g) was dissolved in tetrahydrofuran (100 mL) in a nitrogen atmosphere, and lithium aluminum hydride (2.5 M, 18.97 mL) was slowly added dropwise at 0 °C. The reaction mixture was stirred at 25 °C for 16 h. After the reaction was completed as monitored by TLC (petroleum ether:ethyl acetate = 3:1, potassium permanganate for color development), $H_2O$ (2 mL), 15% NaOH (6 mL) and $H_2O$ (2 mL) were added successively to the reaction mixture at 0 °C to quench the reaction, and then anhydrous magnesium sulfate was added. The mixture was stirred for 30 min and filtered, and the filtrate was concentrated to give intermediate **10-002** (3.70 g).

$^1$H NMR: (400 MHz, Chloroform-d)$\delta$ 4.44 (s, 1H), 4.32 (s, 1H), 3.63 (s, 2H), 1.73 (s, 6H).

**Step 3: synthesis of intermediate 10-1:**

Oxalyl chloride (5.41 g) was dissolved in dichloromethane (40 mL) in a nitrogen atmosphere, and a solution of DMSO (6.66 g) in dichloromethane (20 mL) was slowly added dropwise at -78 °C. The reaction mixture was stirred at -78 °C for 0.5 h. Then, a solution of **10-002** (3.70 g) in dichloromethane (20 mL) was slowly added dropwise, and after the addition was completed, the reaction mixture was stirred at -78 °C for 1 h. Then, a solution of triethylamine (17.3 g) in dichloromethane (20 mL) was slowly added dropwise. The reaction mixture was slowly warmed to room temperature and stirred at room temperature for 16 h. After the reaction was completed as monitored by TLC (petroleum ether:ethyl acetate = 4:1, potassium permanganate for color development), the reaction mixture was washed with saturated brine (20 mL). The organic phase was dried and concentrated to give intermediate **10-1**, which was directly used in the next step.

**Synthesis of intermediate 11-1:**

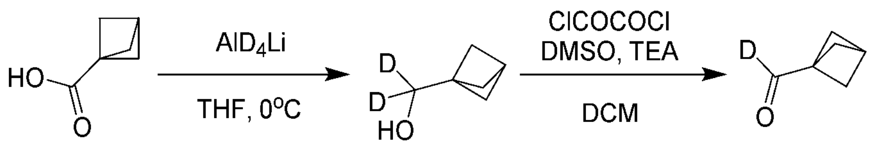


**11-001** **11-002** **11-1**

**Step 1: synthesis of intermediate 11-002**

**11-001** (3.00 g) was dissolved in tetrahydrofuran (50 mL) in a nitrogen atmosphere, and deuterated lithium aluminum hydride (1.70 g) was added slowly at 0 °C. The reaction mixture was stirred at 25 °C for 16 h. After the reaction was completed as monitored by TLC (petroleum ether:ethyl acetate = 2:1, potassium permanganate for color development), $H_2O$ (1.5 mL), a 15% aqueous NaOH solution (1.5 mL), and $H_2O$ (4.5 mL) were added successively to the reaction mixture at 0 °C to quench the reaction, and then anhydrous magnesium sulfate was added. The mixture was stirred for 30 min and filtered, and the filtrate was concentrated to give intermediate **11-002** (2.6 g).
$^1$H NMR (400MHz, Chloroform-*d*) $\delta$ 2.56 (s, 1H), 1.81-1.73 (m, 6H).

**Step 2: synthesis of intermediate 11-1**

Oxalyl chloride (5.04 g) was dissolved in dichloromethane (30 mL) in a nitrogen atmosphere, and a solution of DMSO (6.21 g) in dichloromethane (10 mL) was slowly added dropwise at -78 °C. The reaction mixture was stirred at -78 °C for 0.5 h. Then, a solution of **11-002** (2.60 g) in dichloromethane (20 mL) was slowly added dropwise, and after the addition was completed, the reaction mixture was stirred at -78 °C for 1 h. Then, a solution of triethylamine (16.08 g) in dichloromethane (20 mL) was slowly added dropwise. The reaction mixture was slowly warmed to room temperature and stirred at room temperature for 16 h. After the reaction was completed as monitored by TLC (petroleum ether:ethyl acetate = 2:1, potassium permanganate for color development), the reaction mixture was washed with saturated brine (20 mL).The organic phase was dried to give intermediate **11-1** (2.50 g), which was directly used in the next step.

**Synthesis of intermediate 13-1:**

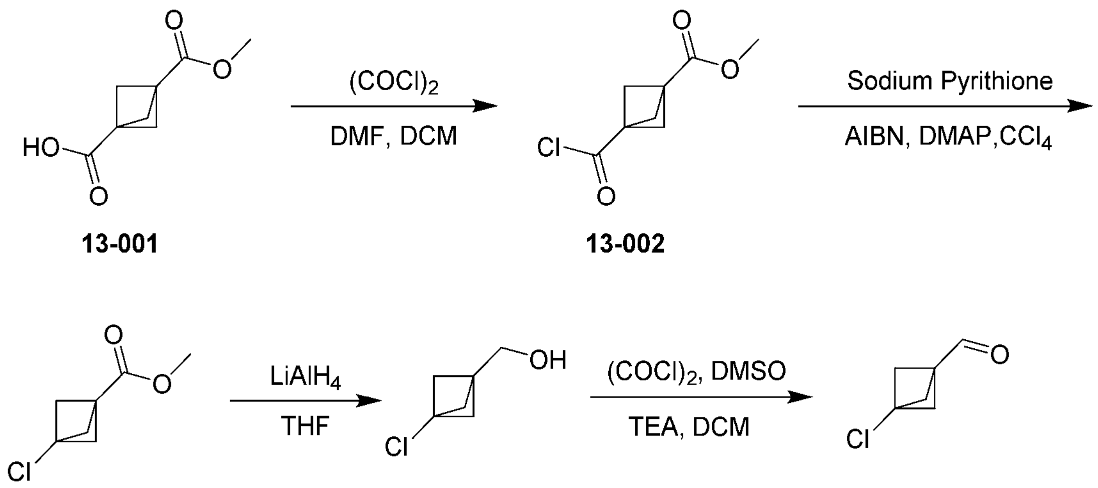


**13-001** **13-002**

**13-003** **13-004** **13-1**

**Step 1: synthesis of intermediate 13-002**

**13-001** (10.0 g) and oxalyl chloride (15.1 g) were dissolved in dichloromethane (100 mL) in a nitrogen atmosphere, and N,N-dimethylformamide (200 mg) was slowly added dropwise at 0 °C. The reaction mixture was stirred at 25 °C for 6 h, and then the reaction was completed. The organic phase was concentrated to give crude intermediate **13-002** (11.0 g), which was directly used in the next step.

**Step 2: synthesis of intermediate 13-003**

Sodium pyrithione (11.0 g), azobisisobutyronitrile (581 mg), and 4-dimethylaminopyridine (144 mg) were dissolved in carbon tetrachloride (100 mL) in a nitrogen atmosphere. The mixture was stirred at 80 °C for 1 h, and then a solution of **13-002** (11.1 g, 59.00 mmol in 50 mL of $CCl_4$ ) was slowly added dropwise. The mixture was stirred at 80 °C for 6 h, and then the reaction was completed. Water (100 mL) was added to quench the reaction, and the mixture was diluted with dichloromethane (50 mL). After liquid separation, the aqueous phase was extracted twice with dichloromethane (100 mL $\times$ 2), and the organic phases were combined, dried, and concentrated to give a crude product. The crude product was

purified by silica gel column chromatography (petroleum ether/ethyl acetate = 5/1) to give intermediate **13-003** (8.00 g).

**Step 3: synthesis of intermediate 13-004**

**13-003** (1.00 g) was dissolved in tetrahydrofuran (10 mL) in a nitrogen atmosphere, and the reaction mixture was cooled to 0 °C. A solution of lithium aluminum hydride in tetrahydrofuran (2.5 M, 2.5 mL) was slowly added dropwise. The reaction mixture was stirred at 0 °C for 3 h, and then the reaction was completed. Water (10 mL) was added to quench the reaction, and ethyl acetate (10 mL) was added for dilution. The phases were separated. The aqueous phase was extracted twice with ethyl acetate (30 mL × 2). The organic phases were combined, washed with saturated brine (30 mL), dried, and concentrated to give crude intermediate **13-004** (800 mg), which was directly used in the next step.

**Step 4: synthesis of intermediate 13-1**

Oxalyl chloride (1.15 g) was dissolved in dichloromethane (20 mL) in a nitrogen atmosphere, and the reaction mixture was cooled to -78 °C. Dimethyl sulfoxide (1.41 g) was slowly added dropwise, and the mixture was stirred for 30 min. Then, **13-004** (800 mg) was slowly added dropwise, and the mixture was stirred at -78 °C for 1 h. Triethylamine (3.66 g) was then added dropwise, and the mixture was warmed to 25 °C. The reaction mixture was stirred at 25 °C for 4 h, and then the reaction was completed. Water (10 mL) was added to quench the reaction, and the phases were separated. The aqueous phase was extracted twice with dichloromethane (10 mL), and the organic phases were combined, washed with saturated brine, and dried to give intermediate **13-1** (788 mg), which was directly used in the next step.

**Example 1: (*R*)-1-(1*H*-benzo[*d*]imidazol-5-yl)-4-(bicyclo[1.1.1]pentan-1-yl)azetidin-2-one**

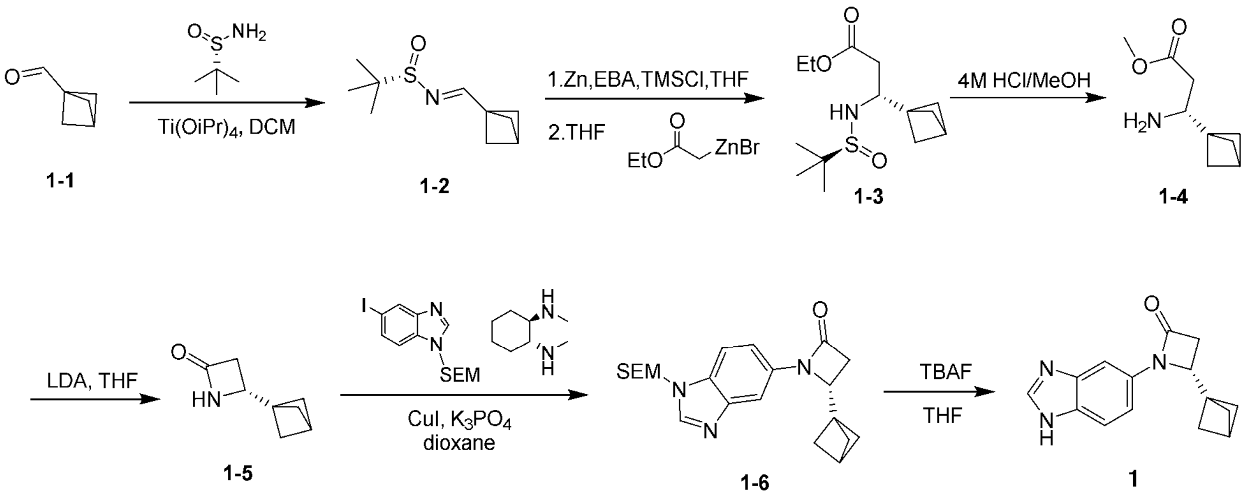


**Step 1: synthesis of intermediate 1-2**

**Compound 1-1** (2.50 g), (S)-butylsulfinamide (3.15 g), and tetraisopropyl titanate (14.78 g) were dissolved in dichloromethane (100 mL) in a nitrogen atmosphere, and the reaction mixture was stirred at 25 °C for 16 h. After the reaction was completed, a saturated aqueous sodium bicarbonate solution (100 mL) was added, and the mixture was stirred. After the solid was filtered, followed by extraction with ethyl acetate (100 mL × 2). The organic phase was dried and concentrated to give a crude product. The crude product was purified by silica gel column chromatography (gradient: 0-10% ethyl acetate/petroleum ether) to give intermediate **1-2** (2.60 g).

$^1$H-NMR(400 MHz, $CDCl_3$) $\delta$ 7.98-7.90 (m, 1H), 2.62-2.53 (m, 1H), 2.08 (s, 6H), 1.23-1.18 (m, 9H).

**Step 2: synthesis of intermediate 1-3**

Zinc powder (5.12 g) was dissolved in tetrahydrofuran (50 mL) in a nitrogen atmosphere, and trimethylsilyl chloride (425 mg) was added thereto. The reaction mixture was stirred at 25 °C for 30 min. The reaction mixture was warmed to 40 °C, and ethyl bromoacetate (6.54 g) was slowly added dropwise thereto. The mixture was stirred at 40 °C for 1 h to give ethoxycarbonylmethylzinc bromide. Intermediate **1-2** (2.60 g) was dissolved in tetrahydrofuran (50 mL), and

ethoxycarbonylmethylzinc bromide was slowly added dropwise at 25 °C. The mixture was stirred at 25 °C for 1 h, and then the reaction was completed. A saturated ammonium chloride solution (100 mL) was added to the reaction mixture to quench the reaction, followed by extraction with ethyl acetate (100 mL × 3). The organic phase was dried over anhydrous sodium sulfate, filtered, and concentrated. The crude product was purified by silica gel column chromatography (gradient: 0-25% ethyl acetate/petroleum ether) to give intermediate **1-3** (3.70 g).

$^1$H-NMR(400 MHz, $CDCl_3$) $\delta$ 4.20-4.12 (m, 3H), 3.65-3.60 (m, 1H), 2.73-2.63 (m, 1H), 2.61-2.48 (m, 2H), 1.73-1.70 (m, 6H), 1.30-1.27 (m, 3H), 1.24 (s, 9H).

**Step 3: synthesis of intermediate 1-4**

Intermediate **1-3** (1.80 g) was dissolved in a solution of hydrogen chloride in methanol (4 M, 40 mL), and the reaction mixture was stirred at 25 °C for 16 h. After the reaction was completed as detected by LCMS, the reaction mixture was concentrated, and the residue was dissolved in water (40 mL), followed by extraction with ethyl acetate (40 mL × 3). The aqueous phase was adjusted to pH 7-8 with a saturated sodium bicarbonate solution and extracted with ethyl acetate (50 mL × 4). The organic phases were combined, dried over anhydrous sodium sulfate, filtered, and concentrated to give intermediate **1-4** (1.20 g).

$^1$H-NMR(400 MHz, $CDCl_3$) $\delta$ 3.71(s, 3H), 3.25-3.24(m, 1H), 2.58-2.43 (m, 2H), 2.27-2.23(m, 1H), 1.73-1.66(m, 6H).

**Step 4: synthesis of intermediate 1-5**

Intermediate **1-4** (150 mg) was dissolved in tetrahydrofuran (1.5 mL) in a nitrogen atmosphere, and a solution of lithium diisopropylamide in THF (2 M, 2.46 mL) was slowly added dropwise at -78 °C over 5 min. The mixture was stirred at -78 °C for 10 h, and then the reaction was completed. A saturated aqueous ammonium chloride solution (5 mL) was added to quench the reaction, and the reaction mixture was extracted twice with ethyl acetate (20 mL × 2). The organic phases were combined, dried over anhydrous sodium sulfate, filtered, and concentrated. The crude product was purified by silica gel column chromatography (petroleum ether:ethyl acetate = 2:1) to give intermediate **1-5** (40.0 mg).

$^1$H-NMR(400 MHz, $CDCl_3$) $\delta$ 5.82 (s, 1H), 3.68-3.62(m, 1H), 2.98-2.93(m, 1H), 2.71-2.63 (m, 1H), 2.62-2.57 (m, 1H), 1.82-1.72 (m, 6H).

**Step 5: synthesis of intermediate 1-6**

Intermediate **1-5** (40.0 mg) was dissolved in dioxane (2 mL) in a nitrogen atmosphere, and 2-[(5-iodobenzimidazol-1-yl)methoxy]ethyltrimethylsilane (120 mg), cuprous iodide (22.2 mg), trans-(1R,2R)-N,N'-dimethyl-1,2-cyclohexanediamine (16.6 mg), and potassium phosphate (185 mg) were added. The mixture was warmed to 80 °C and stirred for 2 h. After the reaction was completed, water (20 mL) and ethyl acetate (10 mL) were added to the reaction mixture for extraction. The organic phase was washed with saturated brine (10 mL × 2), dried over anhydrous sodium sulfate, filtered, and concentrated. The crude product was purified by silica gel column chromatography (gradient: 0-30% tetrahydrofuran/petroleum ether) to give intermediate **1-6** (110 mg).

MS m/z (ESI): = 384.1 $[M+H]^+$.

**Step 6: synthesis of compound 1**

Intermediate **1-6** (140 mg) was dissolved in tetrabutylammonium fluoride (1.85 mL, 1 M in THF), the mixture was stirred at 60 °C for 2 h, and then the reaction was completed. The reaction mixture was concentrated under reduced pressure. Ethyl acetate (50 mL) was added to the crude product, and then the mixture was washed with water (10 mL × 4) and saturated brine (10 mL). The organic phase was dried over anhydrous sodium sulfate, filtered, and concentrated. The crude product was purified by preparative high performance liquid chromatography (column: Phenomenex Gemini NX, 150 × 30 mm, 5 μm; mobile phases: [phase A: water (0.05% ammonia water v/v); phase B: acetonitrile]; gradient elution: mobile phase B from 17% to 57%) to give compound **1** (18.1 mg).

$^1$H-NMR(400 MHz, $CDCl_3$) $\delta$ 8.03 (s, 1H), 7.79 (d, *J* = 1.9 Hz, 1H), 7.64 (d, *J* = 8.7 Hz, 1H), 7.34-7.11 (m, 1H), 4.19-4.13 (m, 1H), 3.18-3.04 (m, 1H), 2.84-2.72 (m, 1H), 2.53 (s, 1H), 1.80 (s, 6H). $^1$H-NMR (400MHz, DMSO-$d_6$) $\delta$ 12.39 (s, 1H), 8.17 (s, 1H), 7.60-7.51 (m, 2H), 7.24 (d, *J* = 8.4 Hz, 1H), 4.30-4.24 (m, 1H), 3.15-3.04 (m, 1H), 2.84-2.68 (m, 1H), 2.49 (s, 1H), 1.74 (s, 6H).

MS m/z (ESI): = 254.1 $[M+H]^+$.

Referring to the synthetic method of Example **1,** compound **1-1** was replaced with the starting materials in the table below to synthesize the following compounds:

| Compound | Starting material | Structure | MS m/z (ESI) | $^1$H-NMR |
|---|---|---|---|---|
| **2** | 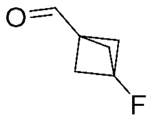 | 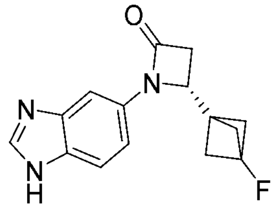 | 271.8 [M+H]$^+$ | $^1$H-NMR (400MHz, DMSO-$d_6$) $\delta$ 12.40 (s, 1H), 8.20 (s, 1H), 7.75-7.13 (m, 3H), 4.66-4.60 (m, 1H), 3.33-3.18 (m, 1H), 2.84-2.81 (m, 1H), 2.04 (d, $J$ = 2.6 Hz, 6H). |
| **3** | 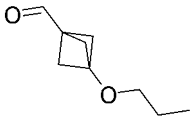 **3-1** | 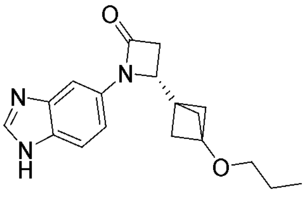 | 312.2 [M+H]$^+$ | $^1$H-NMR (400MHz, DMSO-$d_6$) $\delta$ 12.39 (s, 1H), 8.17 (s, 1H), 7.56 (d, $J$ = 2.0 Hz, 2H), 7.22 (s, 1H), 4.54-4.46 (m, 1H), 3.28 (t, $J$ = 6.7 Hz, 2H), 3.21- 3.09 (m, 1H), 2.83-2.69 (m, 1H), 1.80 (s, 6H), 1.56-1.34 (m, 2H), 0.81 (t, $J$ = 7.4 Hz, 3H). |
| **4** | 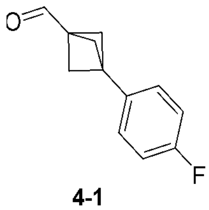 **4-1** | 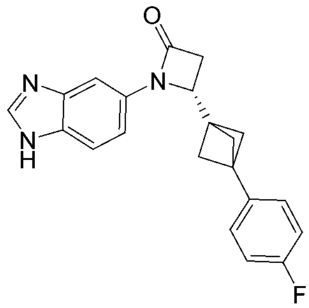 | 348.1 [M+H]$^+$ | $^1$H-NMR (400MHz, DMSO-$d_6$) $\delta$ 12.59-12.22 (m, 1H), 8.27-8.13 (m, 1H), 7.68-7.49 (m, 2H), 7.43-7.16 (m, 3H), 7.13-7.03 (m, 2H), 4.44-4.42 (m, 1H), 3.23-3.11 (m, 1H), 2.86-2.82 (m, 1H), 1.98 (s, 6H). |
| **5** | 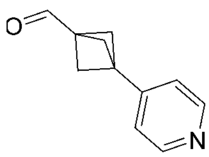 **5-1** | 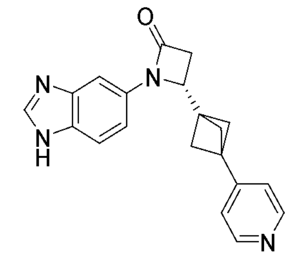 | 331.2 [M+H]$^+$ | $^1$H-NMR (400MHz, DMSO-$d_6$) $\delta$ 12.46-12.40 (m, 1H), 8.49-8.43 (m, 2H), 8.19 (s, 1H), 7.73-7.50 (m, 2H), 7.39-7.17 (m, 3H), 4.51-4.40 (m, 1H), 3.23-3.13 (m, 1H), 2.87-2.83 (m, 1H), 2.03 (s, 6H). |
| **6** | 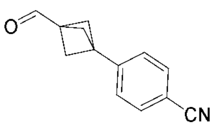 **6-1** | 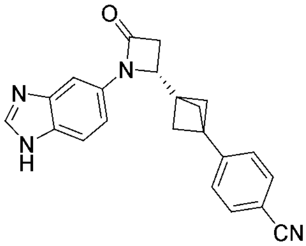 | 355.1 [M+H]$^+$ | $^1$H-NMR (400MHz, DMSO-$d_6$) $\delta$ 8.70 (s, 1H), 7.76-7.69 (m, 4H), 7.42-7.37 (m, 3H), 4.51-4.50 (m, 1H), 3.21-3.19 (m, 1H), 2.29-2.86 (m, 1H), 2.05 (s, 6H). |
| **7** | 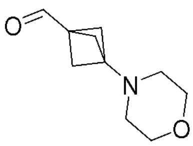 **7-1** | 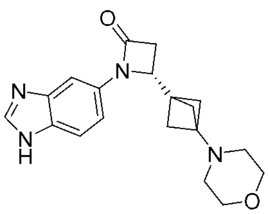 | 339.2 [M+H]$^+$ | $^1$H-NMR (400MHz, DMSO-$d_6$) $\delta$ 7.49-6.80 (m, 4H), 4.59-4.36 (m, 1H), 3.60-3.50 (m, 4H), 3.21-3.12 (m, 1H), 2.79-2.75 (m, 1H), 2.34-2.33 (m, 4H), 1.68 (s, 6H). |

(continued)

| Compound | Starting material | Structure | MS m/z (ESI) | $^1$H-NMR |
|---|---|---|---|---|
| **8** | 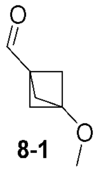 **8-1** | 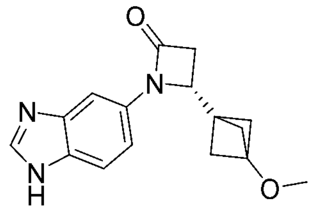 | 284.3 $[M+H]^+$ | $^1$H-NMR (400MHz, DMSO-$d_6$) $\delta$ 12.45 (s, 1H), 8.21 (s, 1H), 7.58 (s, 2H), 7.32-7.16 (m, 1H), 4.57-4.49 (m, 1H), 3.22-3.11 (m, 4H), 2.82-2.72 (m, 1H), 1.80 (s, 6H). |
| **9** | 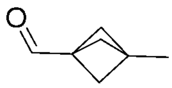 **9-1** | 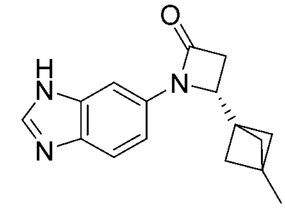 | 268.2 $[M+H]^+$ | $^1$H-NMR (400MHz, DMSO-$d_6$) $\delta$ 12.41 (s, 1H), 8.22 (s, 1H), 7.58-7.07 (m, 3H), 4.30-4.28 (m, 1H), 3.11-3.06 (m, 1H), 2.73-2.70 (m, 1H), 1.59 (s, 6H), 1.11 (s, 3H). |
| **10** | 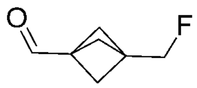 **10-1** | 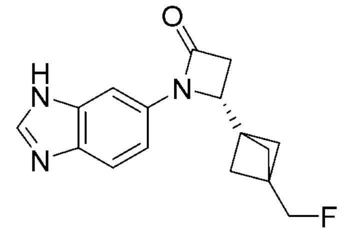 | 286.2 $[M+H]^+$ | $^1$H-NMR (400MHz, DMSO-$d_6$) $\delta$ 12.47 (s, 1H), 8.20 (s, 1H), 7.62-7.49 (m, 2H), 7.25 (d, *J* = 8.4 Hz, 1H), 4.41-4.29 (m, 3H), 3.15-3.10 (m, 1H), 2.81-2.76 (m, 1H), 1.70 (s, 6H). |
| **11** | 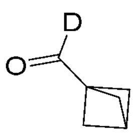 **11-1** | 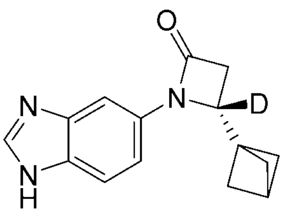 | 255.1 $[M+H]^+$ | $^1$H NMR (400 MHz, DMSO-$d_6$) $\delta$ 12.40 (s, 1H), 8.17 (s, 1H), 7.76-7.43 (m, 2H), 7.24 (d, *J* = 8.4 Hz, 1H), 3.09 (d, *J* = 14.8 Hz, 1H), 2.75 (d, *J* = 14.8 Hz, 1H), 2.49 (s, 1H), 1.75 (s, 6H). |
| **12** | 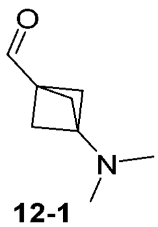 **12-1** | 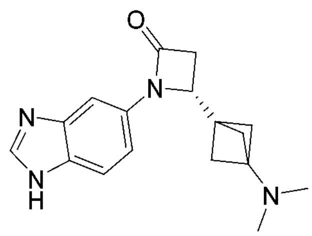 | | |
| **13** | 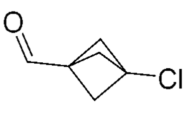 **13-1** | 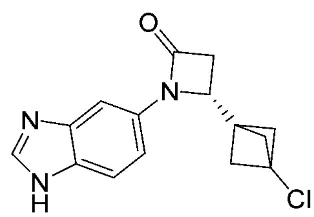 | 288.1 $[M+H]^+$ | $^1$H NMR (400 MHz, DMSO-$d_6$) $\delta$ 12.48 (s, 1H), 8.13 (s, 1H), 7.57 (s, 2H), 7.20 (d, *J* = 8.1 Hz, 1H), 4.61-4.45 (m, 1H), 3.25-3.07 (m, 1H), 2.94-2.80 (m, 1H), 2.14 (s, 6H). |

**Example 14: (*R*)-4-(bicyclo[1.1.1]pentan-1-yl)-1-(imidazo[1,2-*a*]pyridin-7-yl)azetidin-2-one**

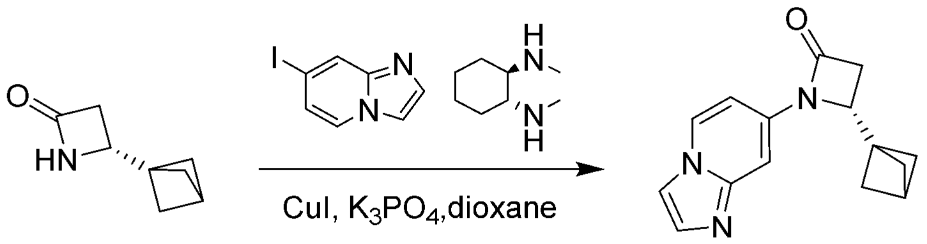


1-5 14

Intermediate **1-5** (50.0 mg) was dissolved in dioxane (2 mL) in a nitrogen atmosphere, and 7-iodoimidazo[1,2-*a*]pyridine (97.0 mg), cuprous iodide (27.7 mg), (1R,2R)-N,N'-dimethyl-1,2-cyclohexanediamine (20.7 mg), and potassium phosphate (232 mg) were added. The mixture was warmed to 80 °C and stirred for 2 h. After the reaction was completed, water (20 mL) and ethyl acetate (10 mL) were added to the reaction mixture for extraction. The organic phase was washed with saturated brine (10 mL × 2), dried over anhydrous sodium sulfate, filtered, and concentrated to give a crude product. The crude product was purified by preparative high performance liquid chromatography (Phenomenex Gemini NX, 150 × 30 mm, 5 μm; mobile phases: [phase A: water (0.5% ammonia water v/v); phase B: acetonitrile]; gradient elution: mobile phase B from 12% to 52%) to give compound **14** (33.8 mg).

$^1$H NMR (400 MHz, DMSO-$d_6$) δ 8.60 (s, 1H), 8.00-7.50 (m, 2H), 7.24-7.15 (m, 2H), 4.34-4.33 (m, 1H), 3.21-3.16 (m, 1H), 2.84-2.80 (m, 1H), 2.50 (s, 1H), 1.79 (s, 6H).

MS m/z (ESI): 254.2 [M+H]$^+$.

**Example 15: (*R*)-1-(1*H*-benzo[*d*]imidazol-6-yl)-4-(bicyclo[1.1.1]pentan-1-yl)-3-methylazetidin-2-one**

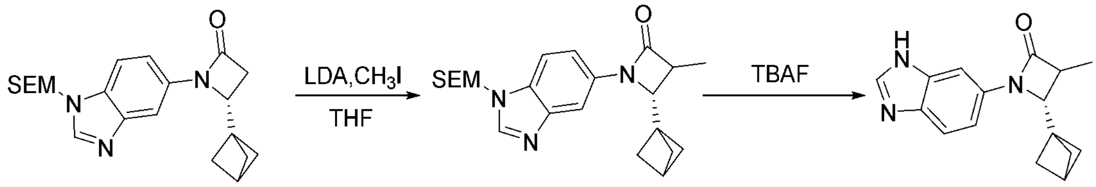


1-6 15-1 15

**Step 1: synthesis of intermediate 15-1**

Intermediate **1-6** (600 mg) was dissolved in tetrahydrofuran (10 mL) in a nitrogen atmosphere, and LDA (1.17 mL, 2 M in THF) was slowly added dropwise at -78 °C. After the dropwise addition was completed, the reaction mixture was stirred at -78 °C for 1 h. Then, iodomethane (244 mg) was added dropwise to the reaction mixture, the reaction mixture was stirred at -78 °C for 1 h, and then the reaction was completed. A saturated aqueous ammonium chloride solution was added to the reaction mixture to quench the reaction, and the aqueous phase was extracted with ethyl acetate (10 mL × 3). The organic phases were combined, washed once with saturated brine (10 mL), dried over anhydrous sodium sulfate, filtered, and concentrated under reduced pressure. The residue was purified by column chromatography (dichloromethane/-methanol gradient elution) to give intermediate **15-1** (50 mg).

MS m/z (ESI): 398.30 [M+H]$^+$.

**Step 2: synthesis of compound 15**

Intermediate **15-1** (50.0 mg) was dissolved in a solution of tetrabutylammonium fluoride in tetrahydrofuran (5.0 mL, 1 M) in a nitrogen atmosphere, the reaction mixture was stirred at 60 °C for 1 h, and then the reaction was completed. The reaction mixture was concentrated under reduced pressure, and the residue was purified by preparative high performance liquid chromatography (Phenomenex Gemini NX, 150 × 30 mm, 5 μm; mobile phases: [phase A: water (0.5% ammonia water v/v); phase B: acetonitrile]; gradient elution: mobile phase B from 50% to 80%) to give the title **compound 15** (15.0 mg).

MS m/z (ESI): 268.2 [M+H]$^+$.

$^1$H NMR (400 MHz, DMSO-$d_6$) δ 8.17 (s, 1H), 7.61-7.51 (m, 2H), 7.23 (d, *J* = 8.7 Hz, 1H), 3.90 (d, *J* = 2.2 Hz, 1H), 3.08-2.98 (m, 1H), 1.76 (s, 6H), 1.25 (d, *J* = 7.6 Hz, 3H).

**Example 16: Synthesis of (*S*)-2-(1*H*-benzo[*d*]imidazol-5-yl)-3-(bicyclo[1.1.1]pentan-1-yl)-2-azaspiro[3.3]heptan-1-one**

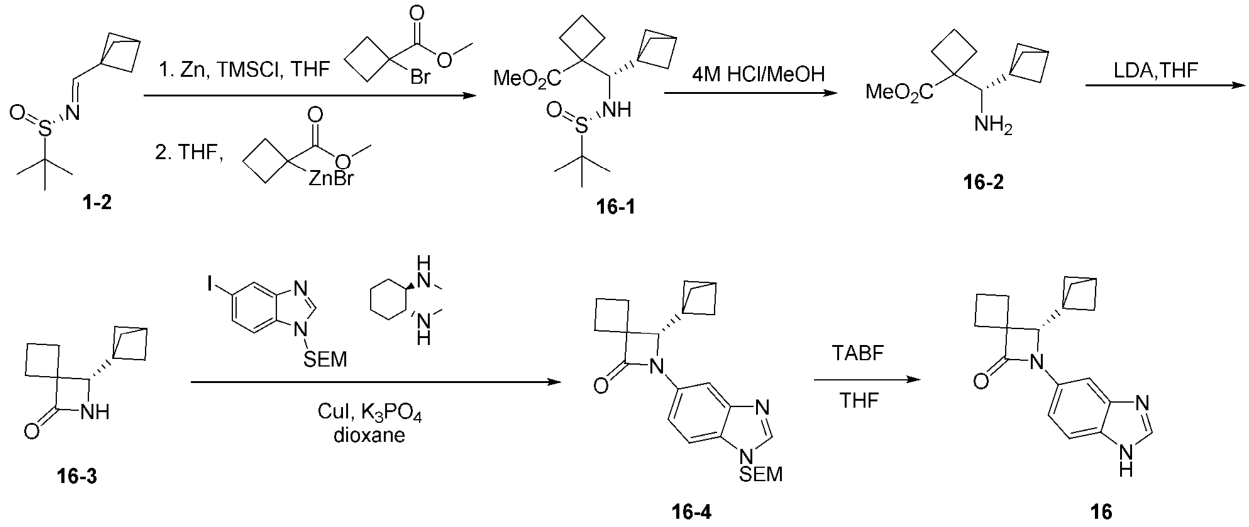


The title **compound 16** was prepared in the same manner as the synthetic method of Example **1**, except that ethyl bromoacetate in step **2** was replaced with methyl 1-bromocyclobutane-1-carboxylate. MS m/z (ESI): 294.2 $[M+H]^+$.

$^1$H NMR (400 MHz, DMSO-$d_6$) δ 8.17 (s, 1H), 7.60-7.49 (m, 2H), 7.21 (d, *J* = 8.6 Hz, 1H), 4.08 (s, 1H), 2.59-2.52 (m, 1H), 2.46 (s, 1H), 2.42-2.32 (m, 1H), 2.27-2.16 (m, 2H), 2.06-1.97 (m, 1H), 1.94-1.69 (m, 7H).

**Example 17: (*R*)-4-(bicyclo[1.1.1]pentan-1-yl)-1-(2-methyl-1*H*-benzo[*d*]imidazol-5-yl)azetidin-2-one**

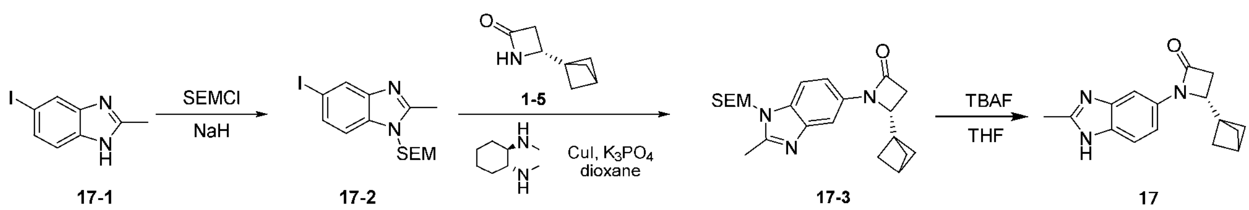


**Step 1: synthesis of intermediate 17-2:**

Intermediate **17-1** ( 3.00 g) was dissolved in N,N-dimethylformamide (30 mL), and the solution was placed in an ice-water bath. Sodium hydride (60% in oil, 522 mg) was slowly added. After stirring for 1 h, 2-(trimethylsilyl)ethoxymethyl chloride (2.27 g) was slowly added dropwise. The mixture was stirred at 25 °C for 2 h, and then the reaction was completed. A saturated aqueous ammonium chloride solution (30 mL) was added to quench the reaction, the reaction mixture was diluted with ethyl acetate (30 mL), and the phases were separated. The aqueous phase was extracted twice with ethyl acetate, and the organic phases were combined, dried, and concentrated to give a crude product. The crude product was purified by silica gel column chromatography (petroleum ether/ethyl acetate = 1/1) to give intermediate **17-2** (3.00 g).

For step **2** and step **3**, reference was made to the synthetic method in step **5** and step **6** of Example **1**, except that 2-[(5-iodobenzimidazol-1-yl)methoxy]ethyltrimethylsilane in step **5** was replaced with **17-2**, thus preparing the title **compound 17** in the same manner.

$^1$H-NMR (400 MHz, DMSO-$d_6$) δ 12.16 (s, 1H), 7.45-7.37 (m, 2H), 7.15 (d, *J* = 8.7 Hz, 1H), 4.28-4.21 (m, 1H), 3.10-3.05 (m, 1H), 2.76-2.71 (m, 1H), 2.50-2.47 (m, 4H), 1.73 (s, 6H).

MS m/z (ESI): = 268.2 $[M+H]^+$.

**Example 18: Synthesis of (4*S*)-1-(1*H*-benzo[*d*]imidazol-6-yl)-4-(bicyclo[1.1.1]pentan-1-yl)-3-fluoroazetidin-2-one**

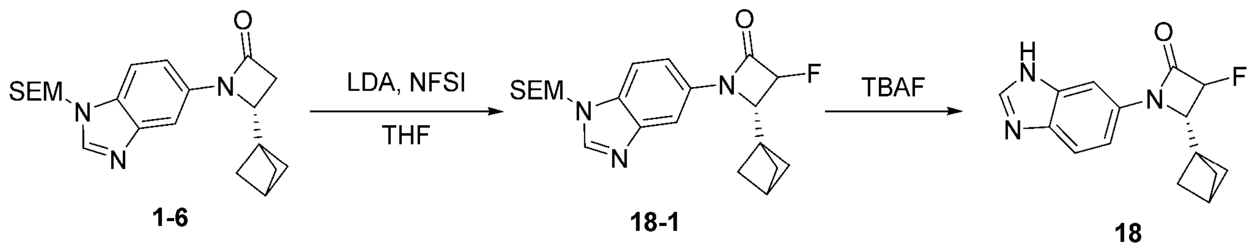


**1-6** **18-1** **18**

**Step 1: synthesis of 18-1**

Intermediate **1-6** (500 mg) was dissolved in tetrahydrofuran (10 mL) in a nitrogen atmosphere, and LDA (2 M in THF, 2 mL) was slowly added dropwise at -70 ° C in a nitrogen atmosphere. The reaction mixture was stirred at that temperature for 30 min. A solution of *N*-fluorobenzenesulfonimide (492 mg) in THF (2 mL) was slowly added dropwise. The mixture was stirred at -70 °C for 1 h, and then the reaction was completed. A saturated aqueous ammonium chloride solution (20 mL) was added to quench the reaction, the reaction mixture was diluted with ethyl acetate, and the phases were separated. The aqueous phase was extracted twice with ethyl acetate, and the organic phases were combined, dried, and concentrated to give a crude product. The crude product was purified by silica gel column chromatography (petroleum ether/ethyl acetate = 1/1) to give intermediate **18-1** (170 mg).

MS m/z (ESI): = 402.30 $[M+H]^+$

**Step 2: synthesis of compound 18**

**18-1** (50.0 mg) was dissolved in a solution of tetrabutylammonium fluoride (1 M in THF, 1.25 mL) in a nitrogen atmosphere, the mixture was stirred at 60 °C for 2 h, and then the reaction was completed. The reaction mixture was concentrated, and the concentrate was purified by silica gel column chromatography (dichloromethane/methanol = 10/1) to give the title **compound 18** (15.0 mg).

$^1$H-NMR (400 MHz, Methanol-$d_4$) $\delta$ 8.19 (s, 1H), 7.68 (d, *J* = 1.9 Hz, 1H), 7.63 (d, *J* = 8.6 Hz, 1H), 7.38 (s, 1H), 5.42 (dd, *J* = 55.1, 1.4 Hz, 1H), 4.41 (dd, *J* = 12.1, 1.3 Hz, 1H), 3.30 (t, *J* = 1.7 Hz, 6H), 2.50 (s, 1H);

$^{19}$F-NMR (376 MHz, Methanol-$d_4$) $\delta$ -198.82.

MS m/z (ESI): = 272.10 $[M+H]^+$

**Test for Biological Activity and Related Properties**

**Unless otherwise stated, the experimental methods used in the following experimental examples are conventional methods. Unless otherwise stated, the materials, reagents, and the like used are commercially available materials and reagents.**

**Experimental Example 1: Inhibition of Human QPCT/QPCTL Enzyme Activity by Examples of the Present Disclosure**

The inhibition of human QPCT/QPCTL enzyme activity by the compounds was assessed using **SensoLyte® Green Glutaminyl Cyclase Activity Assay Kit.**

1. **Experimental reagents, consumables, and instruments**

| Reagent | Brand | Cat. No. |
|---|---|---|
| SensoLyte® Green Glutaminyl Cyclase Activity Assay Kit | Anaspec | AS-72230 |
| Human QPCTL | Sino Biological | NXS6-1 |

(continued)

| Consumables | Brand | Cat. No. |
|---|---|---|
| 384-well, flat bottom, black plate | Greiner | 781900 |
| **Instrument** | **Brand** | **Model** |
| Low-speed centrifuge | Eppendorf | 5810R |
| Water-jacket thermostatic incubator | Bluepard | GHP-9160 |
| Multi-functional microplate reader | Molecular Devices | SpectraMax I3X |
| Microplate shaker | Thermo | 88880024 |

2. **Experimental method:**

0.5 mM QPCT enzyme substrate was diluted to 5 μM with a buffer at a ratio of 1:100; 50 μg/mL QPCT enzyme was diluted to 125 ng/mL with a buffer and placed on ice for later use; QPCT developer was diluted with a buffer at a ratio of 1:250 and placed on ice for later use (the QPCT enzyme substrate, the QPCT enzyme, the buffer, and the QPCT developer were all from the kit). The working solutions of the test compounds were serially 6-fold diluted from an initial concentration of 200 μM to 7 concentration points in total. In the 75 μL reaction system, the initial working concentration of the test compound was 13.3 μM, and the final concentration of DMSO was 0.3%. 20 μL of prepared QPCT enzyme solution, 5 μL of test compound, and 25 μL of QPCT enzyme substrate were successively added to each well of a 384-well plate. The mixture was shaken on a microplate shaker for 1 min, centrifuged at 400 g for 1 min, and incubated at 37 °C for 30 min. Then, 25 μL of QPCT developer was added to each well, and the mixture was sufficiently mixed using a shaker for 1 min, centrifuged at 400 g for 1 min, and incubated at 37 °C for another 60 min. After the incubation was completed, the fluorescence values were read using a microplate reader, with parameter settings as follows: Ex/Em = 490 nm/520 nm. The calculation formula for the inhibition rate of the compounds on QPCT enzyme activity is: inhibition rate (inhibition%) = $(RFU_{no\ treatment} - RFU_{treated}) \div (RFU_{no\ treatment} - RFU_{background}) \times 100\%$. Curves were plotted using GraphPad Prism 9 software with the compound concentration (nM) as the abscissa and the enzyme activity inhibition rate (%) as the ordinate. Four-parameter fitting was performed, and the $IC_{50}$ values for the inhibition of the QPCT enzyme activity by the compounds were calculated. The specific results are shown in Table 1.

**The SensoLyte® Green Glutaminyl Cyclase Activity Assay Kit was appropriately adjusted by replacing the QPCT enzyme in it with human QPCTL enzyme (Sino Biological, Cat. No. NXS6-1) (the concentration of the working solution was kept unchanged)** for determining the $IC_{50}$ values for the inhibition of human QPCTL enzyme activity by compounds. The other experimental reagents, instruments, preparation of working solutions, operation steps, and data processing are the same as those in the human QPCT enzyme activity assay method. The specific results are shown in Table 1.

Table 1. Test results for inhibitory activity of the compounds of the present disclosure against human QPCT/QPCTL enzyme

| Compound | $IC_{50}$(nM) | |
|---|---|---|
| | QPCT | QPCTL |
| 1 | 39.4 | - |
| 2 | 169.7 | - |
| 3 | 52.4 | 6.5 |
| 4 | 10.7 | 3.9 |
| 5 | 8.5 | - |
| 6 | 19.1 | - |
| 7 | 36.8 | 25.1 |
| 8 | 166 | - |
| 9 | 37.3 | |
| 10 | 58.9 | - |
| 11 | 42.5 | - |

(continued)

| Compound | $IC_{50}$(nM) | |
|---|---|---|
| | QPCT | QPCTL |
| 13 | 73.2 | - |
| "-" represents not tested. | | |

**Experimental Example 2: Determination of Membrane Permeability and Transport Characteristics of the Compounds of the Present Disclosure**

The membrane permeability and transport characteristics of the compound of the present disclosure were determined using the following method.

The experimental procedures were as follows:

1. MDCKII-MDR1 (Netherlands Cancer institute) cell culturing

1) Preparation of transport buffer (Hanks balanced salt solution containing 25 mM HEPES, pH 7.4): 5.958 g of 4-hydroxyethylpiperazine ethanesulfonic acid and 0.35 g of $NaHCO_3$ were accurately weighed and dissolved in 900 mL of pure water, and then 100 mL of 10× HBSS was added. The mixture was sufficiently stirred, adjusted to a pH of 7.4, and filtered.
2) Preparation of MDCKII-MDR1 cell culture medium: 10% FBS, 1% Pen Strep Liquid (Gibco), 1% GlutaMAXTM-I (Gibco), and 400 μg/mL G418 (Solarbio) were added to a MEM culture medium (Gibco).
3) Cells were cultured with a T-75 flask in a 5% $CO_2$ incubator at 37 °C. The culture medium was discarded when cell density reached 80-90%. The cells were rinsed with 5 mL of PBS, and then 1.5 mL of Trypsin/EDTA (Gibco) was added. The mixture was incubated at 37 °C for 5-10 min in an incubator until the cells shed like quicksand. Finally, the Trypsin/EDTA was neutralized with an FBS-containing culture medium.
4) The cell suspension was centrifuged at 120 × g for 10 min, and the supernatant was discarded.
5) The cells were resuspended in a cell subculture medium from Step 2, and the cell suspension was adjusted to a density of $1.56 \times 10^6$ cells/mL.

2. MDCKII-MDR1 cell seeding

1) 75 μL of culture medium was added to each Transwell (Corning Corporation) chamber, and 25 mL of culture medium was added to the lower chamber; the plate was preheated in 5% $CO_2$ incubator at 37 °C for 1 h.
2) 50 μL of cell suspension was added to each preheated Transwell chamber at a final density of $5.45 \times 10^5$ cells/$cm^2$.
3) The cells were cultured for 4-8 days. The culture medium was replaced every other day, and the culture medium was replaced within 24 h after the initial seeding. The culture medium must be replaced the day before the experiment.

3. Evaluation of integrity of monolayer cell membrane

1) The cells fused and differentiated after 4-8 days of culturing and were ready for a transport experiment.
2) The resistance of the monolayer membrane was measured using a resistance detector, and the resistance of each well was recorded.
3) After measurement, the Transwell culture plate was re-incubated.
4) The TEER value was calculated:

$$\text{TEER value} = \text{TEER}\ (\Omega)\ \text{measurement value} \times \text{membrane area}\ (\text{cm}^2)$$

The resistance of the monolayer cell membrane < 42 $\Omega\ cm^2$ indicates that a monolayer cell membrane has poor integrity and cannot be used for experiments.

4. Transport experiment

1) A 10 mM stock solution of the compound of the present disclosure or the positive control compound (metoprolol, prazosin, and imatinib) was diluted with DMSO to obtain a 0.2 mM stock solution, and the 0.2 mM stock solution was then diluted with a transport buffer to obtain a 1 μM working solution of the compound of the present disclosure or the positive control compound.
2) The MDCKII-MDR1 cell plate was taken out from the incubator, and the Transwell culture plate was then washed twice with the pre-heated transport buffer and incubated at 37 °C in an incubator for 30 min.
3) To determine the transport rate of the compound from the apical side to the basolateral side (A → B), 125 μL of the compound working solution was added to the Transwell chamber (apical side), and immediately 50 μL of sample was taken from the apical side and added to 200 μL of a stop solution containing an internal standard to stop the transport, serving as an initial apical side sample. At the same time, 235 μL of the transport buffer was added to the receiver side (basolateral side). The sample in the experiment was in duplicate.
4) To determine the transport rate of the compound from the basolateral side to the apical side (B → A), 285 μL of the compound working solution was added to the receiver side (basolateral side), and immediately 50 μL of sample was taken from the basolateral side and added to 200 μL of a stop solution containing an internal standard to stop the transport, serving as an initial basolateral side sample. At the same time, 75 μL of the transport buffer was added to the Transwell chamber (apical side). The sample in the experiment was in duplicate.
5) The cell culture plate was incubated in a $CO_2$ incubator at 37 °C for 2 h.
6) After the transport experiment was completed, 50 μL of sample was taken from the donor side (i.e., the apical side for the A → B direction and the basolateral side for the B → A direction) and added to 200 μL of a stop solution containing an internal standard to stop the transport. 50 μL of sample was taken from the receiver side (i.e., the basolateral side for the A → B direction and the apical side for the B → A direction) and added to 200 μL of a stop solution containing an internal standard. The mixture was vortexed at 1000 rpm for 10 min and centrifuged at 3,220 g for 30 min. 100 μL of the supernatant was transferred to a sample injection plate, and 100 μL of ultrapure water was added. The mixture was sufficiently mixed for LC-MS/MS analysis.
7) Fluorescence values were measured after the transport experiment was completed. A 10 mM lucifer yellow (Sigma) stock solution was prepared with water and then diluted to 100 μM with the transport buffer. 100 μL of the lucifer yellow solution was added to the Transwell chamber (apical side), and 300 μL of the transport buffer was added to the basolateral side. The plate was incubated in a $CO_2$ incubator at 37 °C for 30 min. 80 μL of solution was taken from the basolateral side to a 96-well plate, and the fluorescence value of the cells was measured by using a microplate reader (Infinite® 200 PRO multi-functional microplate reader) at an excitation wavelength of 485 nm and an emission wavelength of 530 nm (to assess the integrity of the membrane).

The fluorescence leakage value (Leakage (%) or LY (%)) of the MDCKII-MDR1 monolayer cell membrane was calculated using the following formula:

$$\text{LY}(\%) = \{I_{\text{acceptor}}\times 0.3/(I_{\text{acceptor}}\times 0.3 + I_{\text{donor}}\times 0.1)\} \times 100\%$$

$I_{\text{acceptor}}$ refers to the fluorescence density of the receiver side (0.3 mL), and $I_{\text{donor}}$ refers to the fluorescence density of the donor side (0.1 mL). LY% > 1.0% indicates that a monolayer cell membrane has poor integrity and the corresponding results will be excluded from the evaluation.

The peak areas of the compound on the donor side and the receiver side were measured. The apparent permeability coefficient ($P_{\text{app}}$, in cm/s) and the efflux ratio (ER) of the compounds were calculated:

$$P_{\text{app}} = \{V_{\text{A}} \times [drug]_{acceptor}/(Area \times incubation\ time \times [drug]_{initial\ dono}\}$$

$V_{\text{A}}$ refers to the volume of the solution at the receiver side (0.235 mL for A → B, and 0.075 mL for B → A); Area refers to the area of the membrane of the Transwell-96-well plate (0.143 $cm^2$); incubation time refers to the time for incubation (in s).

$$EffluxRatio = \frac{P_{app(B-A)}}{P_{app(A-B)}}$$

$P_{app\ (B-A)}$ refers to the apparent permeability coefficient from the basolateral side to the apical side; $P_{app\ (A-B)}$ refers to the apparent permeability coefficient from the apical side to the basolateral side. The calculated apparent permeability coefficients and efflux ratios of the compounds of the present disclosure are shown in Table 2.

Table 2. Apparent permeability coefficients and efflux ratios of the compounds of the present disclosure

| Compound | $P_{app}$ (A-B) ($10^{-6}$, cm/s) | $P_{app}$ (B-A) ($10^{-6}$, cm/s) | Efflux ratio |
|---|---|---|---|
| 1 | 38.5 | 39.4 | 1.0 |
| 2 | 20.2 | 41.3 | 2.0 |
| 3 | 18.7 | 47.3 | 2.5 |
| 4 | 6.5 | 7.4 | 1.2 |
| 8 | 11.0 | 37.2 | 3.4 |
| 9 | 22.7 | 21.6 | 1.0 |
| 10 | 17.7 | 32.3 | 1.8 |
| 13 | 18.5 | 34.4 | 1.9 |
| 14 | 35.4 | 35.9 | 1.0 |

**Experimental Example 3: Inhibitory Effect of the Compounds of the Present Invention on CYP1A2, CYP2C9, CYP2C19, CYP2D6, and CYP3A4 Enzyme Activity**

The inhibition of CYP1A2, CYP2C9, CYP2C19, CYP2D6, and CYP3A4 enzyme activity by the compounds of the present disclosure was determined using the following method.

The experimental procedures were as follows:

1. Preparation of 100 mM phosphate-buffered saline (PBS): 7.098 g of $Na_2HPO_4$ was weighed, added to 500 mL of pure water, and dissolved by sonication to obtain solution A. 3.400 g of $KH_2PO_4$ was weighed, added to 250 mL of pure water, and dissolved by sonication to obtain solution B. The solution A was placed in a stirrer, and the solution B was slowly added until the pH reached 7.4, thus obtaining the 100 mM PBS buffer.
2. A 10 mM NADPH solution was prepared with the 100 mM PBS buffer. A 10 mM stock solution of the compound of the present disclosure was diluted with DMSO to obtain a compound working solution at a 200× concentration (10000, 3333.3, 1111.1, 370.37, 123.46, 41.15, 13.7, and 0 μM). The positive inhibitor stock solution was diluted with DMSO to obtain a positive inhibitor working solution at a 200× concentration (sulfaphenazole: 1000, 300, 100, 30, 10, 3, and 0 μM; quinidine/ketoconazole: 100, 30, 10, 3, 1, 0.3, and 0 μM; α-naphthoflavone: 50, 15, 5, 1.5, 0.5, 0.15, and 0 μM; nootkatone: 6000, 1800, 600, 180, 60, 18, and 0 μM). Substrate working solutions (8000 μM phenacetin, 1000 μM diclofenac, 8000 μM S-mephenytoin, 1000 μM dextromethorphan, and 400 μM midazolam) at a 200× concentration were prepared with water, acetonitrile, or acetonitrile/methanol.
3. 1 μL of 20 mg/mL liver microsome solution, 1 μL of substrate working solution, 1 μL of compound working solution, and 177 μL of PBS buffer were taken, mixed sufficiently, and placed in a 37 °C water bath for pre-incubation for 10 min. 1 μL of positive inhibitor working solution, instead of the compound working solution, was added to the positive control group. At the same time, 10 mM NADPH solution was pre-incubated together in the 37 °C water bath for 10 min. After 10 min, 20 μL of NADPH was added to each well to initiate the reaction. The mixture was incubated at 37 °C for 15 min (CYP1A2), 15 min (CYP2C9), 60 min (CYP2C19), 15 min (CYP2D6), or 15 min (CYP3A4). All incubated samples were in duplicate. After incubation for the corresponding period of time, 400 μL of icy methanol containing an internal standard was added to all samples to stop the reaction. The resulting mixture was vortexed for sufficient mixing and centrifuged at 4000 rpm and 4 °C for 30 min. After the centrifugation was completed, 100 μL of the supernatant was transferred to a sample injection plate, and 200 μL of ultrapure water was added. The mixture was sufficiently mixed for LC-MS/MS analysis.

The values were calculated using Excel XLfit 5.3.1.3 to obtain $IC_{50}$ values of the compounds of the present disclosure against CYP1A2, CYP2C9, CYP2C19, CYP2D6, and CYP3A4, as shown in Table 3.

Table 3. $IC_{50}$ values of the compounds of the present disclosure against CYP1A2, CYP2C9, CYP2C19, CYP2D6, and CYP3A4

| Compou nd | CYP1A2 (μM) | CYP2C9 (μM) | CYP2C19 (μM) | CYP2D6 (μM) | CYP3A4 (μM) |
|---|---|---|---|---|---|
| 1 | >50 | 48.7 | >50 | - | >50 |
| 2 | >50 | >50 | >15 | >50 | >50 |
| 3 | >50 | >50 | - | >50 | - |
| 7 | - | >50 | - | >50 | >50 |
| 8 | >50 | >50 | 34.8 | >50 | >50 |
| 9 | >50 | 29 | - | >50 | 41.7 |
| 10 | >50 | 31.2 | 31 | >50 | >15 |
| 13 | >50 | 46 | >15 | >50 | >50 |
| 14 | - | >50 | 36.9 | 29.6 | >50 |
| "-" represents not tested. | | | | | |

**Experimental Example 4. Determination of Metabolic Stability of Compounds of the present Disclosure in Liver Microsomes**

The experimental procedures were as follows:
1. Preparation of 100 mM phosphate-buffered saline (PBS): 7.098 g of $Na_2HPO_4$ was weighed, added to 500 mL of pure water, and dissolved by sonication to obtain solution A. 3.400 g of $KH_2PO_4$ was weighed, added to 250 mL of pure water, and dissolved by sonication to obtain solution B. The solution A was placed in a stirrer, and the solution B was slowly added until the pH reached 7.4, thus obtaining the 100 mM PBS buffer.
2. Preparation of reaction system
The reaction system was prepared as follows:

| Reagent | Stock solution concentration | Volume | Final concentration of system |
|---|---|---|---|
| Liver microsomes (Corning) | 20 mg/mL | 5 μL | 0.5 mg/mL |
| Phosphate-buffered saline | 100 mM | 173 μL | 100 mM |

3. The reaction system was pre-incubated in a 37 °C water bath for 10 min. 20 μL of 10 mM NADPH solution (NADPH was dissolved by 100 mM phosphate-buffered saline) was added to the reaction system with a final concentration of NADPH being 1 mM. 20 μL phosphate-buffered saline was used instead of the NADPH solution to serve as the negative control. The negative control was used to exclude the effect of chemical stability of the compound itself.
4. The reaction was initiated by adding 2 μL of 100 μM compound of the present disclosure and verapamil, the positive control compound, to the reaction system with a final concentration of the compound being 1 μM.
5. At 0.5, 15, 30, 45, and 60 min, 25 μL of the incubation sample was taken out, and the reaction was stopped with 4-fold glacial acetonitrile solution containing an internal standard. The samples were centrifuged at 4000 rpm for 15 min. After the centrifugation was completed, 50 μL of the supernatant was transferred to a sample injection plate, and 150 μL of ultrapure water was added. The mixture was sufficiently mixed for LC-MS/MS analysis.

All data were calculated using Microsoft Excel software. The peak area was obtained by extracting an ion chromatogram. Through linear fitting of the natural logarithm of the parent drug elimination percentage versus time, the *in vitro* half-life ($t_{1/2}$) of the parent drug was determined.

The *in vitro* half-life ($t_{1/2}$) was calculated through the slope (k):

$$\textit{in vitro}\ t_{1/2} = 0.693/k$$

The $t_{1/2}$ values calculated using the above formula are shown in Table 4.

Table 4. Half-life ($t_{1/2,}$ min) of the compounds of the present disclosure in liver microsomes of different species

| Compound | Half-life ($t_{1/2,}$ min) (human) | Half-life ($t_{1/2,}$ min) (rat) | Half-life ($t_{1/2,}$ min) (mouse) |
|---|---|---|---|
| 1 | 1812 | 63.2 | 75.9 |
| 2 | 1916 | 325 | 495 |
| 4 | 164.4 | 93.0 | 61.2 |
| 7 | 315.7 | - | 325.4 |
| 8 | 2130 | - | - |
| 9 | 79.3 | - | 160.8 |
| 10 | 413.1 | - | 416.2 |
| 13 | 988 | 249 | 246 |
| 14 | 156.3 | 36.3 | - |
| "-" represents not tested. | | | |

## Claims

**1.** A compound of formula (A-I) or a stereoisomer thereof or a pharmaceutically acceptable salt thereof,

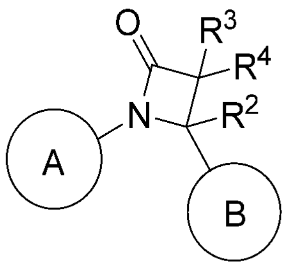

(A-I)

wherein

ring A is selected from

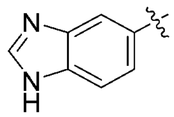

or

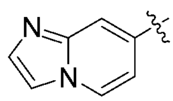

,

wherein the

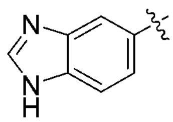

or

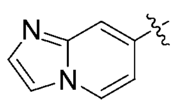

is optionally substituted with $R^5$;
$R^5$ is selected from D, halogen, CN, $NH_2$, OH, $C_1$-$C_6$ alkyl, $C_1$-$C_6$ alkoxy, $C_1$-$C_6$ alkylamino, $C_1$-$C_6$ haloalkyl, or $C_1$-$C_6$ deuterated alkyl;
ring B is selected from

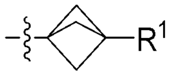 ;

$R^1$ is selected from H, halogen, OH, $NH_2$, SH, CN, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ cycloalkyl, or 4- to 10-membered heterocyclyl, wherein the OH, $NH_2$, SH, $C_1$-$C_6$ alkyl, $C_2$-$C_6$ alkenyl, $C_2$-$C_6$ alkynyl, $C_6$-$C_{10}$ aryl, 5- to 10-membered heteroaryl, $C_3$-$C_6$ cycloalkyl, or 4- to 10-membered heterocyclyl is optionally substituted with $R^{1a}$; $R^{1a}$ is selected from halogen, CN, OH, $NH_2$, or $C_1$-$C_6$ alkyl, wherein the OH, $NH_2$, or $C_1$-$C_6$ alkyl is optionally substituted with $R^{1b}$;
$R^{1b}$ is selected from halogen or $C_1$-$C_6$ alkyl;
$R^2$ is selected from H, D, or $C_1$-$C_6$ alkyl;
$R^3$ and $R^4$ are independently selected from H, D, halogen, OH, $C_1$-$C_6$ alkyl, $C_2$-$C_6$ alkenyl, $C_2$-$C_6$ alkynyl, $C_3$-$C_6$cycloalkyl, or 4- to 7-membered heterocyclyl, wherein the OH, $C_1$-$C_6$ alkyl, $C_2$-$C_6$alkenyl, $C_2$-$C_6$ alkynyl, $C_3$-$C_6$ cycloalkyl, or 4- to 7-membered heterocyclyl is optionally substituted with $R^{3a}$; or $R^3$ and $R^4$, together with the atom to which they are attached, form $C_3$-$C_6$ cycloalkyl or 4- to 7-membered heterocyclyl, wherein the $C_3$-$C_6$ cycloalkyl or 4- to 7-membered heterocyclyl is optionally substituted with $R^{3a}$;
$R^{3a}$ is selected from D, halogen, OH, or $C_1$-$C_6$ alkyl.

**2.** The compound or the stereoisomer thereof or the pharmaceutically acceptable salt thereof according to claim 1, wherein $R^1$ is selected from H, halogen, OH, $NH_2$, $C_1$-$C_6$ alkyl, phenyl, 5- to 6-membered heteroaryl, or 4- to 7-membered heterocyclyl, wherein the OH, $NH_2$, $C_1$-$C_6$ alkyl, phenyl, 5- to 6-membered heteroaryl, or 4- to 7-membered heterocyclyl is optionally substituted with $R^{1a}$; or $R^1$ is selected from H, halogen, OH, $NH_2$, $C_1$-$C_6$ alkyl, phenyl, pyridinyl, or morpholinyl, wherein the OH, $NH_2$, $C_1$-$C_6$ alkyl, phenyl, pyridinyl, or morpholinyl is optionally substituted with $R^{1a}$; or

$R^1$ is selected from H, F, Cl, OH, $NH_2$, $CH_3$,

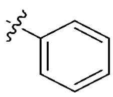, 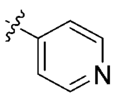, or 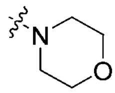,

wherein the OH, $NH_2$, $CH_3$,

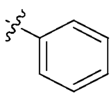, 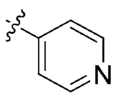, or 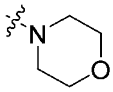

is optionally substituted with $R^{1a}$; or
$R^1$ is selected from H, F,

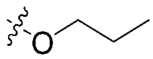, 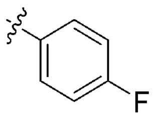, 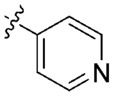, 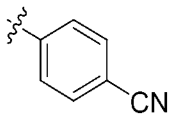, 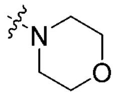,

$CH_3$, $CH_2F$,

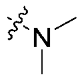,

Cl, or $OCH_3$.

3. The compound or the stereoisomer thereof or the pharmaceutically acceptable salt thereof according to claim 1 or 2, wherein $R^{1a}$ is selected from halogen, CN, or $C_1$-$C_6$ alkyl.

4. The compound or the stereoisomer thereof or the pharmaceutically acceptable salt thereof according to any one of claims 1-3, wherein $R^2$ is selected from H or D.

5. The compound or the stereoisomer thereof or the pharmaceutically acceptable salt thereof according to any one of claims 1-4, wherein $R^3$ and $R^4$ are independently selected from H, halogen, or $C_1$-$C_6$ alkyl, or $R^3$ and $R^4$, together with the atom to which they are attached, form $C_3$-$C_6$ cycloalkyl, wherein the $C_1$-$C_6$ alkyl and $C_3$-$C_6$ cycloalkyl are optionally substituted with $R^{3a}$; or

   $R^3$ and $R^4$ are independently selected from H, F, or methyl, or $R^3$ and $R^4$, together with the atom to which they are attached, form cyclobutyl; or
   $R^3$ and $R^4$ are both H.

6. The compound or the stereoisomer thereof or the pharmaceutically acceptable salt thereof according to any one of claims 1-5, wherein
   $R^5$ is $CH_3$.

7. The compound or the stereoisomer thereof or the pharmaceutically acceptable salt thereof according to any one of claims 1-6, wherein the compound of formula (A-I) is selected from a compound of formula (I) or a stereoisomer thereof or a pharmaceutically acceptable salt thereof:

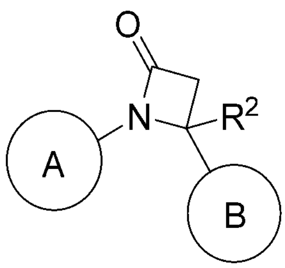

(I)

   wherein ring A, ring B, and $R^2$ are as defined in any one of claims 1-6.

8. The compound or the stereoisomer thereof or the pharmaceutically acceptable salt thereof according to claim 7, wherein

   the compound of formula (A-I) is selected from a compound of formula (II) or a stereoisomer thereof or a pharmaceutically acceptable salt thereof:

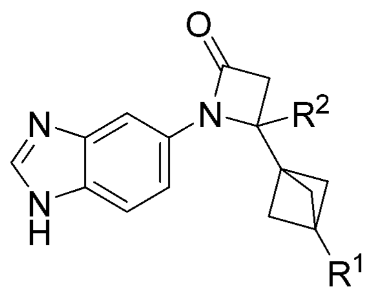

   (II), wherein $R^1$ and $R^2$ are as defined in any one of claims 1-6.

9. The compound or the stereoisomer thereof or the pharmaceutically acceptable salt thereof according to claim 8, wherein

the compound of formula (A-I) is selected from a compound of formula (II-1) or a stereoisomer thereof or a pharmaceutically acceptable salt thereof:

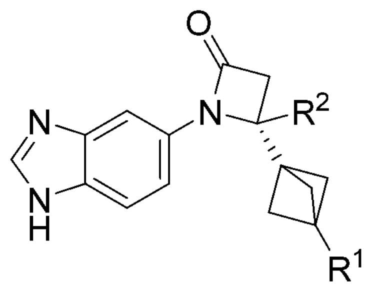

(II-1)

wherein $R^1$ and $R^2$ are as defined in any one of claims 1-6.

**10.** The compound or the stereoisomer thereof or the pharmaceutically acceptable salt thereof according to claim 7, wherein the compound of formula (A-I) is selected from a compound of formula (III) or a stereoisomer thereof or a pharmaceutically acceptable salt thereof:

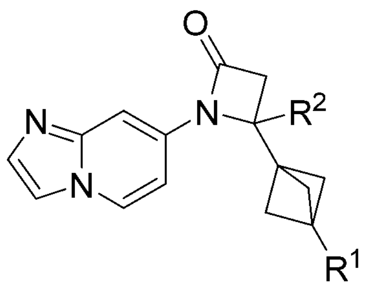

(III)

wherein $R^1$ and $R^2$ are as defined in any one of claims 1-6.

**11.** The compound or the stereoisomer thereof or the pharmaceutically acceptable salt thereof according to claim 10, wherein the compound of formula (A-I) is selected from a compound of formula (III-1) or a stereoisomer thereof or a pharmaceutically acceptable salt thereof:

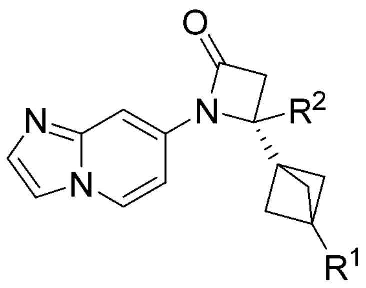

(III-1)

wherein $R^1$ and $R^2$ are as defined in any one of claims 1-6.

**12.** The compound or the stereoisomer thereof or the pharmaceutically acceptable salt thereof according to any one of claims 1-6, wherein the compound of formula (A-I) is selected from the following compounds or stereoisomers thereof or pharmaceutically acceptable salts thereof:

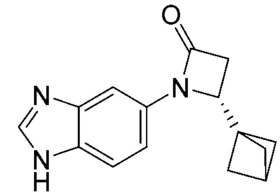

1

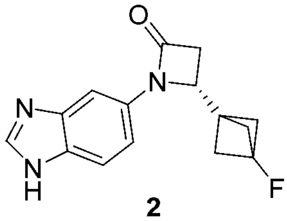

2

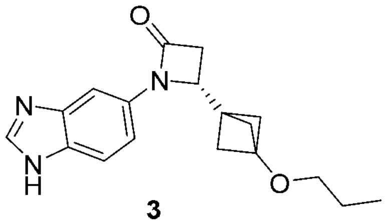

3

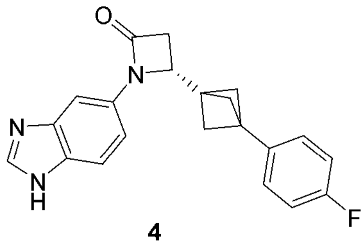

4

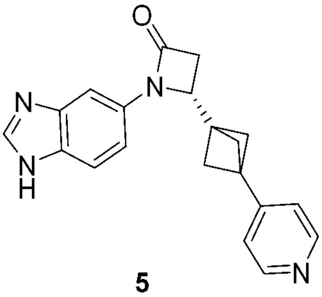

5

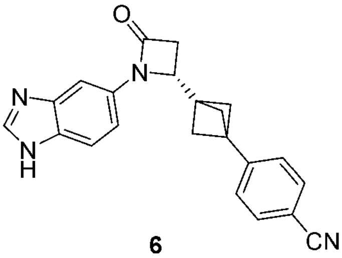

6

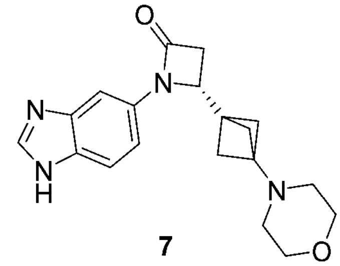

7

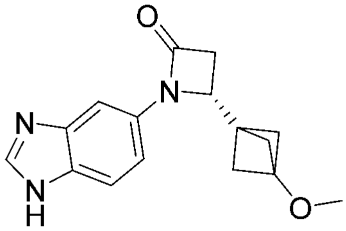

8

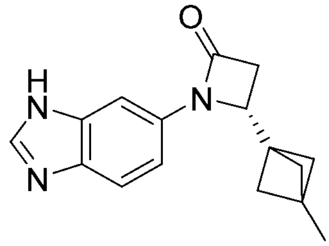

9

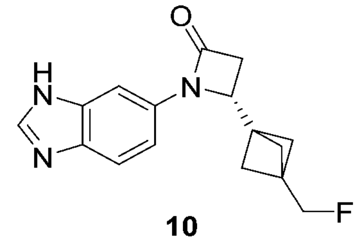

10

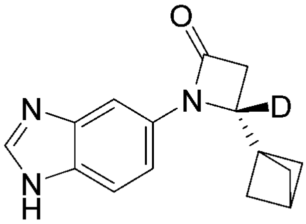

11

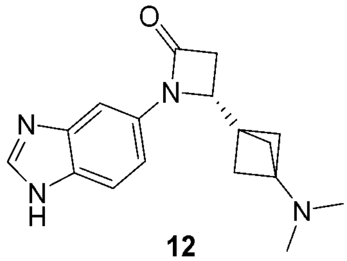

12

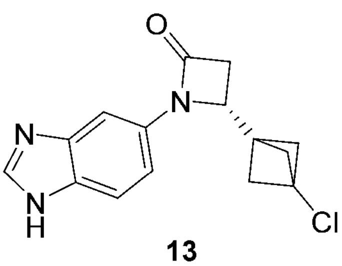

13

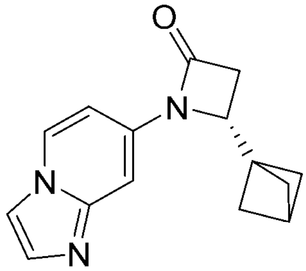

14

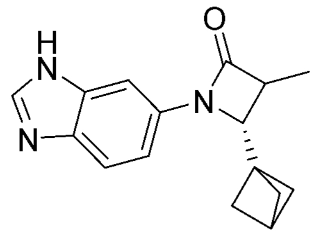

15

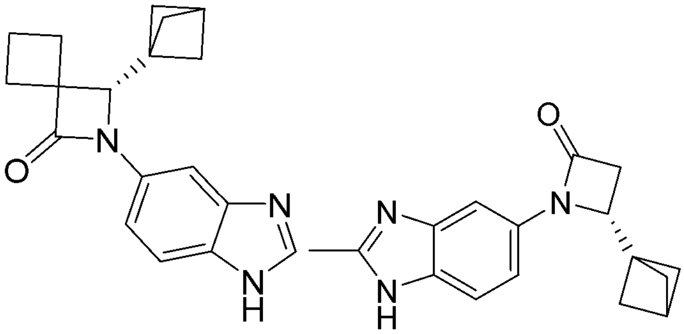

**16** 17 , or

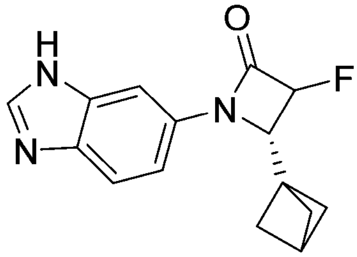

**18** .

13. A pharmaceutical composition, comprising the compound of formula (I) or the stereoisomer thereof or the pharmaceutically acceptable salt thereof according to any one of claims 1-12 and optionally a pharmaceutically acceptable excipient.

14. The compound of formula (I) or the stereoisomer thereof or the pharmaceutically acceptable salt thereof according to any one of claims 1-12 or the pharmaceutical composition according to claim 13 for use in treating or preventing a disease or disorder mediated by QPCT and/or QPCTL in a subject in need thereof.

15. The compound of formula (I) or the stereoisomer thereof or the pharmaceutically acceptable salt thereof or the pharmaceutical composition according to claim 14, wherein the disease or disorder mediated by QPCT and/or QPCTL is a neurodegenerative disease, preferably Alzheimer's disease.

**INTERNATIONAL SEARCH REPORT**

International application No. **PCT/CN2024/121304**

**A. CLASSIFICATION OF SUBJECT MATTER**

C07D403/04(2006.01)i; C07D205/04(2006.01)i; C07D471/04(2006.01)i; C07D487/00(2006.01)i; A61K31/4184(2006.01)i; A61K31/397(2006.01)i; A61P25/28(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, ENTXT, DWPI, CNKI, STN (REGISTRY, CAPLUS, MARPAT), BAIDU: 先声药业, 杨圣伟, 唐锋, 刘乐, 赵晨, 周旻昀, 苯并咪唑, 氮杂环丁烷, 酮, 阿尔茨海默, 谷氨酰胺, 环化酶, QPCT? , alzheime?, glutami?, cyclase?, cyclotransfera?, azetidi?, benz?, imidazo?, structural formula search

**C. DOCUMENTS CONSIDERED TO BE RELEVANT**

| Category* | Citation of document, with indication, where appropriate, of the relevant passages | Relevant to claim No. |
|---|---|---|
| A | WO 2022268179 A1 (INSILICO MEDICINE IP LTD.) 29 December 2022 (2022-12-29) see claims 1, 12, and 96-10,1 and description, table 1 | 1-15 |
| A | CN 111315738 A (PROBIODRUG AG) 19 June 2020 (2020-06-19) see claims 1-15, and description, embodiments 1-31, compounds | 1-15 |

☐ Further documents are listed in the continuation of Box C.

☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 December 2024** | **31 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/121304**

| Patent document cited in search report | Publication date (day/month/year) | Patent family member(s) | Publication date (day/month/year) |
|---|---|---|---|
| WO 2022268179 A1 | 29 December 2022 | AU 2022298746 A1 | 30 November 2023 |
| | | TW 202317549 A | 01 May 2023 |
| | | KR 20240024948 A | 26 February 2024 |
| | | EP 4359405 A1 | 01 May 2024 |
| | | JP 2024523552 A | 28 June 2024 |
| | | CA 3221003 A1 | 29 December 2022 |
| | | US 2023192667 A1 | 22 June 2023 |
| | | US 11834440 B2 | 05 December 2023 |
| | | US 2024116899 A1 | 11 April 2024 |
| CN 111315738 A | 19 June 2020 | CA 3077314 A1 | 04 April 2019 |
| | | CA 3077314 C | 13 July 2021 |
| | | BR 112020005625 A2 | 20 October 2020 |
| | | BR 112020005625 B1 | 24 May 2022 |
| | | KR 20210057206 A | 20 May 2021 |
| | | EA 202190664 A1 | 27 May 2021 |
| | | DK 3461819 T3 | 10 August 2020 |
| | | PL 3461819 T3 | 30 November 2020 |
| | | SG 11202002239 VA | 29 April 2020 |
| | | MX 2020003570 A | 19 November 2021 |
| | | WO 2019063414 A1 | 04 April 2019 |
| | | US 2020223825 A1 | 16 July 2020 |
| | | US 11279690 B2 | 22 March 2022 |
| | | ZA 202001573 B | 28 April 2021 |
| | | EA 202090642 A1 | 14 May 2020 |
| | | EA 037862 B1 | 28 May 2021 |
| | | KR 20200074948 A | 25 June 2020 |
| | | KR 102251645 B1 | 14 May 2021 |
| | | NZ 763312 A | 28 April 2023 |
| | | IL 281216 A | 29 April 2021 |
| | | ES 2812698 T3 | 18 March 2021 |
| | | JP 2020535186 A | 03 December 2020 |
| | | JP 6821860 B2 | 03 February 2021 |
| | | EP 3461819 A1 | 03 April 2019 |
| | | EP 3461819 B1 | 27 May 2020 |
| | | AU 2018340397 A1 | 23 April 2020 |
| | | AU 2018340397 A8 | 30 April 2020 |
| | | AU 2018340397 B2 | 11 June 2020 |
| | | IL 273214 A | 30 April 2020 |
| | | IL 273214 B | 25 March 2021 |
| | | JP 2021073202 A | 13 May 2021 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311273882
- CN 202311736403

### Non-patent literature cited in the description

- **MAEHR**. *J. Chem. Ed.*, 1985, vol. 62, 114-120